(19)

**Europäisches Patentamt**
**European Patent Office**
**Office européen des brevets**

(11) **EP 3 971 909 A1**

(12) **EUROPEAN PATENT APPLICATION**

(43) Date of publication:
**23.03.2022 Bulletin 2022/12**

(21) Application number: **20197251.0**

(22) Date of filing: **21.09.2020**

(51) International Patent Classification (IPC):
**G16H 50/20** *(2018.01)*

(52) Cooperative Patent Classification (CPC):
**G16H 50/20**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Kaiser, Thorsten**
**04275 Leipzig (DE)**

• **Staritzbichler, René**
**04275 Leipzig (DE)**

(72) Inventors:
• **Kaiser, Thorsten**
**04275 Leipzig (DE)**
• **Staritzbichler, René**
**04275 Leipzig (DE)**

(74) Representative: **Maikowski & Ninnemann**
**Patentanwälte Partnerschaft mbB**
**Postfach 15 09 20**
**10671 Berlin (DE)**

(54) **METHOD FOR PREDICTING MARKERS WHICH ARE CHARACTERISTIC FOR AT LEAST ONE MEDICAL SAMPLE AND/OR FOR A PATIENT**

(57) The invention provides a method to predict markers which are characteristic for at least one medical sample and /or for a patient, wherein the method comprises the steps of measuring at least one spectrum of at least one medical sample of a patient with at least one spectroscopic method. According to the method the sample is examined in an untargeted manner. By bioinformatics processing of the measured spectra multiple values which are characteristic for said sample can be calculated, which are predictions for certain markers. The predicted values can be compared with results of conventional routine laboratory medical diagnostic and/or interpreted in the context of reference intervals and/or clinical decision limits. A report including recommended further actions for a user is created and due to the results, further diagnostic steps can be initiated by the user, which allows accelerated and more specific treatment of a patient.

FIG 6

EP 3 971 909 A1

**Description**

The invention provides a method to predict markers which are characteristic for at least one medical sample and /or for a patient, wherein the method comprises the steps of measuring at least one spectrum of at least one medical sample of a patient with at least one spectroscopic method. According to the method the sample is examined in an untargeted manner. By bioinformatics processing of the measured spectra multiple values which are characteristic for said sample can be calculated, which are predictions for certain markers. The predicted values can be compared with results of conventional routine laboratory medical diagnostic and/or interpreted in the context of reference intervals and/or clinical decision limits. A report including recommended further actions for a user is created and due to the results, further diagnostic steps can be initiated by the user, which allows accelerated and more specific treatment of a patient.

For diagnosis, choosing adequate therapy and monitoring the course of the treatment and disease, the majority of patients require laboratory diagnostics. 60 - 70 % of all therapeutic decisions in medicine are based on laboratory diagnostics (Regan & Forsman 2006). Thus, almost 7 billion laboratory tests are performed annually in the USA (Makary & Daniel 2016). The analysis of laboratory medical patient diagnostics is quality-assured at the highest level and is regarded as very precise.

Actually, laboratory diagnostics for medical care is characterized by single biomarker analysis. These parameters are requested individually by physicians according to the suspected diagnosis of the patient. In a first step the sample is usually taken at the clinic or doctor's office and send to the medical laboratory. After arrival at the medical laboratory the requested parameters are analyzed with different methods. These different methods, mainly each for one biomarker, are usually performed in parallel on huge and expensive analyzers. Each biomarker determination is billed separately, which also leads to considerable costs. For each analysis a minimum amount of the medical sample is necessary. The whole amount of required sample volume is the sum of these individual amounts needed for each of the different methods, which sometimes limits the number of tests that can be performed.

The majority of these routine methods in a medical laboratory are highly specific for single biomarkers. As an example, troponin is released from the heart muscle in case of myocardial infarction. It can be detected using highly specific antibodies and a very sensitive detection system in a so-called immune assay. The production of suitable detection antibodies is complex and costly. For myoglobin, another important biomarker if a myocardial infarction is suspected, the detection requires different methods with different detection antibodies. The methods are processed in parallel on large mechanized analyzers in different reaction vials, but are not measured simultaneously in the same assay. The most important methods in a medical laboratory are clinical chemistry reactions, immune assays, clotting assays for coagulation diagnostics and flow cytometry for cell analysis. With the exception of the latter, different methods are used for each individual biomarker. True multiparameter analysis is limited to expensive special diagnostics such as multiplex PCR-Assays for the molecular detection of multiple viral pathogens (up to about 15) at the same time in one assay.

At present, laboratory medical diagnostics is carried out depending on the clinical picture and the medical question according to the principle of the specific request of individual diagnostic parameters (targeted diagnostics). The practitioner must therefore already define a suspected diagnosis at the time of the laboratory request. In addition, he or she must have knowledge of the appropriate diagnostic laboratory medical parameters and select them - a precondition that is not always given.

In current laboratory medical diagnostics, errors regularly occur, especially in the pre-analytical phase of the diagnostic process, which can limit the validity of the diagnosis and lead to incorrect medical consequences (Plebani 2006). Relevant pre-analytical influencing factors and interfering factors are the therapy with different drugs and food supplements (e.g. antibiotics, biotin), dilutions with infusion solutions, analyte concentrations above the measuring range of the method (Hook effect), material inhomogeneities (e.g. air bubbles and clots) as well as sample ageing.

All routine methods of laboratory medicine have so far been specific detection methods for individual biomarkers (single parameter diagnostics), which can at best be partially parallelized on large analysis platforms. Biomarkers are measurable indicators of a biological state or condition. An untargeted, broad, so-called "untargeted" analysis, which could detect many different biomarkers simultaneously, would be of great advantage for fast and cost-efficient diagnostics. Spectrometric methods are of particular interest in this context, since they in principle provide an untargeted analysis approach. However, spectrometric, untargeted methods have considerable limitations. First of all, biomarkers in laboratory medicine are often very complex proteins that require spectra that are difficult to interpret. Due to the complexity of biosamples containing several thousand biomarkers in various physiological concentrations ranging from less than ng/l to several g/l, specific detection and quantification of individual analytes whose spectra overlap during measurement is not possible with diagnostic certainty. A further, fundamental problem with low-concentration biomarkers is the weak signal especially in relation to strong noise. In addition, for example in Raman spectroscopy the substance-specific spectra, which can be measured in dry form and which can be clearly assigned, largely disappear in the noise and fluorescence when the substances are brought into solution.

Attempts have been made to use Raman spectroscopy for performing quantitative analysis of serum and serum ultrafiltrate, thereby predicting the concentration of a small amount of different high concentrated biomarkers (e.g. albumin reference value 35-53g/l). Fitting tools were used to interpret the spectra, in this way it was possible to predict the concentration of the 8 biomarkers with the highest concentrations in blood serum. However, the prediction of biomarkers with low concentration starting from urea acid (reference values ♀ < 61 mg/ml ♂ <82 mg/ml) suffers from high uncertainty, that does not allow to detect low concentrated biomarkers with reasonable precision. Troponin (reference value <14pg/ml), for example, cannot be detected at all by conventional Raman spectroscopy. The study summarizes that prediction accuracy decreases with decreasing concentration of analytes in the sample and thus using Raman spectroscopy for performing quantitative analysis is limited to analytes with a lower concentration limit in the mg/dl range. Investigations of analytes having concentrations below this limit need to be done with a sample preparation like ultrafiltration or have to be done with other methods (Rohleder et al. 2004).

Accordingly, sensitivity and specificity are insufficient in comparison to laboratory-medical targeted diagnostics. As a consequence, untargeted analyses have no importance in current laboratory medical patient diagnostics, especially without complex prior sample preparation.

It is the purpose of the present invention to provide a method to carry out untargeted analytics with medical samples in a highly sensitive manner. The obtained information should be suitable to perform validity and quality checks and to complement conventional targeted routine laboratory medical diagnostic of a medical sample. It also allows a screening diagnostic prior to the specific and sensitive targeted standard diagnostics.

Therefore, the present invention provides a method to predict markers which are characteristic for at least one medical sample and /or for a patient comprising the steps:

- Passing at least one medical sample to at least one spectrometer by a user;
- Measuring at least one spectrum of the at least one medical sample with at least one spectroscopic method;
- Calculating multiple values $m_{pred}$ with the at least one spectrum of the at least one medical sample using multiple optimal predictive models;
- Classifying the calculated values $m_{pred}$ in terms of defined thresholds;
- Creating a report including recommended actions for the user;
- Initiating further diagnostic steps by the user;

wherein the at least one medical sample is examined in an untargeted manner;
wherein the values $m_{pred}$ are predictions of markers, which are characteristic for said at least one medical sample and/or a patient; and
wherein each value $m_{pred}$ is calculated by a separate predictive model.

Further, a device adapted to perform the method steps

- Passing at least one medical sample to at least one spectrometer by a user;
- Measuring at least one spectrum of the at least one medical sample with at least one spectroscopic method;
- Calculating multiple values $m_{pred}$ with the at least one spectrum of the at least one medical sample using multiple optimal predictive models;
- Classifying the calculated values $m_{pred}$ in terms of defined thresholds;
- Creating a report including recommended actions for the user;
- Initiating further diagnostic steps by the user;

is provided, comprising

  ○ At least one spectrometer;
  ○ At least one data processing device;
  ○ At least one user interface.

The method according to the invention or the device according to the invention are used for validity and quality check of conventional targeted routine laboratory medical diagnostic, for performing untargeted diagnostics, to obtain a rapid diagnosis of suspicion and/or to for monitoring of laboratory values and/or clinical features of e.g. a patient at home, in a doctor's practice or in the clinic.

**Detailed description**

The present invention provides a method to predict markers which are characteristic for at least one medical sample and/or for a patient. In one step of the present invention a medical sample is passed to at least one spectrometer by a user.

In context of the invention a user can be generally every person able to operate a spectrometer and able to work with medical samples, these include in particular clinicians, general practitioner and laboratory specialists. Since, at least some spectroscopic methods are implementable in easy operable tools, in one embodiment of the invention also a patient can be a user according to the invention. Medical samples suitable for the present invention are selected from the group comprising blood, blood plasma, blood serum, cerebrospinal fluid (CSF), urine, saliva and exhaled breath condensate.

In one embodiment of the invention one medical sample of one patient is used in the method of the invention. In a further embodiment of the invention more than one medical sample of one patient is used in the method of the invention, wherein the medical samples are of different types, for example blood plasma, urine and CSF could be used. The number of medical samples from one patient is not limited and according to the invention every combination of types of medical samples can be used. This has the advantage, that information contained in different types of medical samples can be evaluated with the present method leading to a more precise prediction.

According to the invention at least one spectrum of the at least one medical sample with at least one spectroscopic method is measured.

In one embodiment of the invention more than one spectrum is measured with the at least one spectroscopic method of the at least one medical sample. Preferably, between two to ten spectra, more preferably between two to five spectra are measured with the at least one spectroscopic method.

In a preferred embodiment of the invention not only one spectrum of the at least one medical sample is measured but a multiple of spectra, wherein each spectrum is measured with another spectroscopic method. Since the physical background of different spectroscopic methods is fundamentally different, the information contained in the spectra of different spectroscopic methods differs as well. The physical background of the spectroscopic method used according to the invention are well known to the person skilled in the art and will not be explained in this context further. Accordingly, spectra obtained by different spectroscopic methods often contain information about the medical sample which complement each other. The combination of spectra obtained by different spectroscopic methods in the predictive model according to the invention therefore significantly increases the quality of the predictive model.

In one embodiment of the invention the method further comprises the step of measuring respectively at least one spectrum of the at least one sample by 1 to 7 further spectroscopic methods, preferably by 1 to 4 further spectroscopic methods.

In one embodiment of the invention the spectroscopic method used in the invention is selected from the group containing Raman spectroscopy, plasmon-enhanced Raman spectroscopy (PERS), including surface-enhanced Raman spectroscopy (SERS), shell-isolated nanoparticle-enhanced Raman spectroscopy (SINERS) and tip-enhanced Raman spectroscopy (TERS), near infra-red spectroscopy (NIR), ultraviolet spectroscopy (UV), visible spectroscopy (VIS), magnetic resonance spectroscopy (MR), terahertz radiation (TR) and mass spectroscopy (MS).

In a preferred embodiment of the invention Raman spectroscopy, IR-spectroscopy and Mass spectroscopy are used in combination to measure spectra of the medical sample. In a most preferred embodiment of the invention Raman spectroscopy and IR-spectroscopy are used in combination to measure spectra of the medical sample.

In a further preferred embodiment of the invention at least two spectra are measured with the same spectroscopic method using different measurement settings. According to the invention measurement settings are defined as settings which are specific for a certain method and which may influence the information obtained in the spectra. For example, in Raman spectroscopy spectra can be obtained with different Raman excitation frequencies and different optical settings or in Nuclear Magnetic Resonance spectroscopy spectra can be obtained for different nuclei, e.g. $^1$H or $^{13}$C or different experimental setups. Analogous to using different spectroscopic method the information contained in the spectra can complement each other and therefore can increase the quality of the prediction.

In a preferred embodiment of the invention Raman spectroscopy is used as one spectroscopic method. In a further preferred embodiment Raman spectroscopy is used with two different Raman excitation frequencies.

Advantageously, most spectroscopic methods need just small amounts of samples to be carried out. Accordingly, in one embodiment of the invention between 1μl and 150μl, preferably between 30μl and 100μl, most preferably 50μl of medical sample are used to measure all spectra used in the method according to the invention. Since medical samples have a limited volume, it is of great benefit that just a small part of the medical sample is necessary to carry out the method according to the invention. It allows a general untargeted screening diagnostic for all samples prior to or in parallel to targeted conventional laboratory diagnostic. Still a dominant part of a medical sample is available to carry out further diagnostics such as routine laboratory medical diagnostic.

Further, according to the invention multiple values $m_{pred}$ are calculated by bioinformatic processing of at least one spectrum of at least one medical sample using a predictive model. The values $m_{pred}$ are predictions for markers which are characteristic for the at least one medical sample or for the status of a patient, wherein these characteristic values are selected from the group comprising laboratory values, clinical features and sample characteristics.

In the context of the invention laboratory values comprise concentrations of single substances and combinations of such, wherein combinations of single concentrations of substances are called scores. Clinical features are selected from the group comprising:

- Patient's sex and age
- Clinical Diagnosis
- Medical complications
- Progress of a disease
- Risk of mortality
- Fasting
- Intake of drugs and food supplements
- Intoxications
- Drug interactions

Further, sample characteristics can be for example the sample-age. Some of these markers have numerical values per se, such as laboratory values. However, a part of the markers, e.g. clinical features and sample characteristics are not represented by numerical values per se. According to the invention the information of these markers is translated into numerical values, e.g. "0:no", "1:yes" or '0:dead', '1:alive'. Hence, every marker has a numerical representation.

Any of the markers, which are reflected in the spectra can be predicted by a value $m_{pred}$, wherein according to the invention $m_{pred}$ is a prediction of a numerical representation of a certain marker. For each $m_{pred}$ predicting a certain marker a separate predictive model is used.

According to the invention a predictive model must be derived for every value $m_{pred}$ which is used to predict a certain marker which is characteristic for the medical sample or for the status of a patient.

According to the invention the optimal predictive model to calculate a value $m_{pred}$ or a vector $m_{pred}$ is generated by a model based or a model-free approach.

In one embodiment of the invention model free learning algorithm, such as ,deep learning', are applied. A successful application of model-free approaches depends on cohorts above 10000 patients being available. In machine learning one distinguishes classification and regression problems. Classifications are assignments to distinct classes such as ,healthy' and 'ill'. Regressions predict continuous range of values such as molecular concentrations. Main classes of machine learning algorithms are linear and logistic regression, artificial neural networks, support vector machines, decision trees, as well as Bayesian networks. The applied method depends therefore on the nature of the marker, whether it is binary, discrete or continuous. In this embodiment artificial intelligence or more precisely supervised machine learning is applied to predict markers from spectral data.

In a further implementation of the invention, predictions of markers are derived in a complex model-based learning approach. Model based means in this context that the algorithm is based on knowledge about the nature of the data, that it is targeted towards solving that specific problem. Learning means that the algorithm contains free parameters, which are adjusted such that the calculated values match the expected values best. The model-based implementation has the advantage to reach correlations above 0.7 for independent verification test data sets when only a medium number of spectra and according marker are available, ranging from 100 to 1000.

According to the invention in one embodiment an optimal predictive model is generated to calculate a value $m_{pred}$ by a method comprising the steps:

1) Passing at least 100 medical samples to at least one spectrometer by a user;
2) Measuring at least one spectrum of each of the at least 100 medical samples with at least one spectroscopic method;
3) Pre-processing all spectra;
4) Reading a value $m_{ref}$ which represents a reference value for $m_{pred}$ for every of the at least 100 medical samples;
5) Dividing all of the pre-processed spectra into a training data set, a test data set and a validation data set;

6) Representing the values $m_{ref}$ which belong to the training data set by a vector $\overrightarrow{m}_{ref}^{train}$, the values $m_{ref}$ which belong to the test data set by a vector $\overrightarrow{m}_{ref}^{test}$ and the values $m_{ref}$ which belong to the validation data set by a vector $\overrightarrow{m}_{ref}^{val}$;

7) Generating a first predictive model to calculate a vector $\overrightarrow{m}_{pred,1}^{train}$ by bioinformatic processing using the spectra of the training data set, wherein the bioinformatic processing comprises the steps of

a. Selection of n frequencies, with $n \in N$;

b. Calculating optimal weighting values for the frequencies selected;

c. Calculating the vector $\vec{m}_{pred,1}^{train}$;

8) Calculating a quality score $d_1^{train}$ utilizing $\vec{m}_{ref}^{train}$ and $\vec{m}_{pred,1}^{train}$;

9) Utilizing the first predictive model to calculate a vector $\vec{m}_{pred,1}^{test}$ using the spectra of the test data set and calculating a quality score $d_1^{test}$ utilizing $\vec{m}_{ref}^{test}$ and $\vec{m}_{pred,1}^{test}$;

10) Generating a further predictive model to calculate a vector $\vec{m}_{pred,2}^{train}$ by bioinformatic processing comprising the steps

a. Selection of n frequencies, with $n \in N$;

b. Calculating optimal weighting values for the frequencies selected;

c. Calculating the vector $\vec{m}_{pred,2}^{train}$; using the spectra of the training data set, wherein the n frequencies selected in step a. are iteratively altered compared to the frequencies selected in any of the steps before;

11) Calculating a quality score $d_2^{train}$ utilizing $\vec{m}_{ref}^{train}$ and $\vec{m}_{pred,2}^{train}$;

12) Utilizing the further predictive model to calculate a vector $\vec{m}_{pred,2}^{test}$ using the spectra of the test data set and calculating a quality score $d_2^{test}$ utilizing $\vec{m}_{ref}^{test}$ and $\vec{m}_{pred,2}^{test}$;

13) Comparing the quality scores and only if $d_2^{train} < d_1^{train}$ and $d_2^{test} < d_1^{test}$ the further predictive model is taken as optimal predictive model and the first predictive model is discarded; if the condition $d_2^{train} < d_1^{train}$ and $d_2^{test} < d_1^{test}$ is not fulfilled the first predictive model is taken as optimal predictive model and the further predictive model is discarded; and renaming the quality scores of the optimal predictive model as $d_{opt}^{train}$ and $d_{opt}^{test}$;

14) Repeating steps 10) to 12) and comparing the quality scores of the optimal predictive model and the further predictive model and only if $d_2^{train} < d_{opt}^{train}$ and $d_2^{test} < d_{opt}^{test}$ the further predictive model is taken as optimal predictive model; if the condition $d_2^{train} < d_{opt}^{train}$ and $d_2^{test} < d_{opt}^{test}$ is not fulfilled the optimal predictive model is unchanged and the further predictive model is discarded; and renaming the quality scores of the optimal predictive model as $d_{opt}^{train}$ and $d_{opt}^{test}$ if necessary;

15) Repeating step 14) for all possible combinations of n frequencies;

16) Utilizing the optimal predictive model to calculate a vector $\vec{m}_{pred}^{val}$ using the spectra of the validation data set;

17) Validating the optimal predictive model by calculating a correlation coefficient and a z-score utilizing the vector $\vec{m}_{pred}^{val}$;

wherein the values of the vector $\vec{m}_{pred}^{train}$ are predictions of the values of the vector $\vec{m}_{ref}^{train}$, the values of the vector $\vec{m}_{pred}^{test}$ are predictions of the values of the vector $\vec{m}_{ref}^{test}$ and the values of the vector $\vec{m}_{pred}^{val}$ are predictions of the values of the vector $\vec{m}_{ref}^{val}$.

In the following the method to generate a predictive model is described in more detail.

Accordingly, after passing at least 100 medical samples to at least one spectrometer at least one spectrum of each medical sample is measured with at least one spectroscopic method. Suitable spectroscopic methods are already described above. One value $m_{ref}$ which is a numerical representation of a certain marker is read for every of the at least 100 medical samples. According to the invention every value $m_{ref}$ of the at least one measured spectrum is assigned to a certain medical sample. Hence it is well known which value $m_{ref}$ and which measured spectra belong to a certain medical sample.

Accordingly, in order to derive a predictive model to calculate a value $m_{pred}$ it requires to have sufficient reference values $m_{ref}$ Accordingly, $m_{ref}$ are characteristic values collected in the medical context, which are used during the derivation and/or optimization of the prediction method. Each of the above mentioned markers which are characteristic for the medical sample or for a patient may serve as reference values $m_{ref}$, and are used for the optimization of an according $m_{pred}$. Hence, a pair $m_{ref}$, $m_{pred}$ relates to the same type of marker which is characteristic for the medical sample or the patient. As already described above, for markers which are not per se numbers, such as the occurrence of a clinical feature, this information is translated into numerical values, e.g. "0:no", "1:yes" or '0:dead', '1:alive'. Accordingly, in a preferred embodiment of the invention $m_{ref}$ is a numerical representation of a marker.

According to the invention at least 100 medical samples are used to derive the predictive model. In a preferred embodiment of the invention the at least 100 medical samples are each from different sources e.g. patients and could have the same nature, e.g. all samples are urea or they could have different nature, e.g. a part is urea and a part is blood plasma. It is quite obvious that also more than 100 medical samples can be used to derive the predictive model. In one embodiment of the invention for deriving a suitable predictive model, at least one spectrum is measured for each of 100 to 100.000 medical samples, preferably at least one spectrum is measured for each of 200 to 50.000 medical samples, most preferably at least one spectrum is measured for each of 400 to 10.000 medical samples.

For each of the at least 100 medical samples at least one spectrum is measured with at least one spectroscopic method. According to the invention more than one spectrum can be measured with the at least one spectroscopic method for each medical sample. Suitable numbers of spectra are already described above. Further, more than one spectra can be measured for each medical sample with at least one spectroscopic method using different measurement settings. Suitable spectroscopic methods are already described above.

Further, at least one spectrum can be measured for each medical sample utilizing different spectroscopic methods and/or different measurement settings. Preferable combinations are already described above.

In one further embodiment of the invention temporal changes in the spectra of the medical sample and/or the background signals of the spectroscopic methods are used in the bioinformatic processing as additional information. Measurements and processing time have an impact on some markers such as biomarkers or other properties of the sample. For example, in Raman spectroscopy a high energy laser beam is applied to the sample, which causes bleaching of some components in the sample. As a consequence, following the time dependence of fluorescence and Raman spectra reveal altered spectral information that add additional information and can improve the predictive model, and thus the quality of the calculation of $m_{pred.}$.

In one further embodiment of the invention, difference spectra are exploited, based on changes in biochemical conditions such as pH and/or physical conditions such as temperature. Each change will lead to differences in the spectra. These changes may add further information and improve the predictive model.

The spectra obtained for each of the at least 100 medical samples together build a set of spectra. Pre-Processing is done for all spectra of the set of spectra in the following way.

Pre-Processing

Firstly, pre-processing is done for every spectrum of the set of spectra comprising the steps of calibration, background filtering and optionally normalization and/or adding of derived spectra.

Background filtering has to be done depending on the spectroscopic method used to measure the spectrum on which background filtering is applied. Generally, background signals are extraneous signals that can be mixed up with the phenomenon to be observed or measured. The phenomenon is well known to persons skilled in the art and background filtering is one of the crucial steps to reveal the information content of the spectrum. Background filtering according to the invention comprises at least one of the following steps:

- subtracting a signal of a reference sample;
- filtering out a background signal by a fitting algorithm;
- applying a noise filter.

Depending on the spectroscopic method used to measure the spectrum on which background filtering should be done, one, two or all steps of background filtering are applied to the spectrum.

Fitting algorithms to filter background signals as well as noise filters are known to the persons skilled in the art. Suitable fitting algorithms can be tuned to which degree they follow local fluctuations of the spectrum. These parameters are often considered the 'stiffness' and 'flexibilty' of the smoothing curve.

Exemplarily the subtraction of a signal of a reference sample and background filtering is explained for a spectrum obtained by Raman Spectroscopy. Especially in Raman Spectroscopy spectral patterns disappear in solution even for substances that may have a clear and distinct spectrum when measured dry. Instead, strong fluorescence and strong noise appear. Noise is the contribution to the spectrum which does not contain any information. This forbids the application of standard methods such as principle component analysis, which rely on large variation in data points being actual information. Thus, the complex bioinformatic procedures according to the invention were developed, including the pre-processing of the spectra.

For a Raman spectrum in the initial step, the signal arising from water in the sample or material of the sample carrier can be removed. This is done by subtracting a signal of a reference sample. The reference sample can be for example water.

In a next step the background signal arising from fluorescence is handled, by filtering out the background signal by a fitting algorithm. The difference between a peak arising from Raman Spectroscopy (a so-called Raman peak) and a peak arising from fluorescence (a so-called fluorescence peak) is their width. Raman peaks have a width of a few wavenumbers, while a fluorescence peak covers a broad range of the spectrum. Therefore, one can filter them out by a fitting algorithm that clings to the part of the spectrum arising from fluorescence and due to its stiffness does not follow local fluctuations or peaks arising from Raman spectroscopy. The fluorescence spectrum is extracted and can be used as a further spectrum obtained by a further method (fluorescence spectroscopy) to increases the quality of the predictive model.

In one embodiment of the invention the fluorescence spectrum extracted from the spectrum obtained by Raman spectroscopy is used as further spectrum in the method of the invention.

Generally, according to the invention a noise filter can be applied. Two averaging methods are usually used. One method averages over a triangular window and the other method averages over a window with a Gaussian shape, having tails in the negative range (Savitzky-Golay-filter).

In one embodiment of the invention noise filtering is applied depending on the value $m_{ref}$ and/or the spectroscopic method used to obtain the spectrum, meaning for some values $m_{ref}$ and/or some spectroscopic methods noise filtering is done while for other values $m_{ref}$ and/or some spectroscopic methods no noise filtering is applied. Also, noise filtered and not noise filtered spectra can be used both together as two spectra for the same value $m_{ref}$.

Optionally the spectra obtained after background filtering are normalized. In one embodiment the spectra obtained after background filtering are normalized.

The normalization of the spectrum can be performed in multiple ways, wherein normalization procedures are well known to persons skilled in the art. For example, normalization can be done using the boundaries of the spectral values as reference or alternatively their mean value and standard deviation. In one embodiment of the invention normalization involves centering the spectra. In a further embodiment of the invention normalization does not involve centering the spectra.

However, normalization is not always beneficial for the method according to the invention, thus in one embodiment of the invention the spectrum is further evaluated using a spectrum which is not normalized.

Further, optionally additional derived spectra are added to the spectrum. Derived spectra are spectra selected from the group comprising:

- spectra which are derived by using other parameters for calibration and/or background filtering and/or normalization;
- spectra which are the elementwise difference, ratio or mean of two or more spectra
- the spectrum which is the mathematical derivative of the at least one spectrum;
- the spectrum which contains the background signal of the at least one spectrum.

For example, in case Raman Spectroscopy was used as spectroscopic method the fluorescence spectrum contained as background signal in the at least one spectrum can be used as derived spectra.

Accordingly, in one embodiment of the invention at least one derived spectrum is added to the at least one spectrum. This has the advantage, that additional spectral content is added for bioinformatic processing without the need for measuring additional spectra. Further, the derived spectra can contain spectral information which complement the information of the measured spectra. In some cases, the spectral information stands out more clearly in a derived spectra in comparison to the measured spectra due to mathematical operations applied to the measured spectra to obtain the derived spectra (e.g. mathematical derivative, differential spectrum).

By pre-processing of the set of spectra a set of pre-processed spectra is obtained.

In a next step the set of pre-processed spectra is divided into a training data set, a test data set and a validation data set, wherein each of the pre-processed spectra is assigned to exactly one of the data sets. For example, 1/3 of the pre-processed spectra can be assigned to the training data set, 1/3 of the pre-processed spectra can be assigned to the test data set and 1/3 of the pre-processed spectra can be assigned to the validation data set. For every of the at least 100 medical samples a value $m_{ref}$ is read which represents a reference value for $m_{pred}$. The values $m_{ref}$ which belong to medical samples whose spectra are assigned to the training data set are represented by a vector $\overrightarrow{m}_{ref}^{train}$. The values $m_{ref}$ which belong to medical samples whose spectra are assigned to the test data set are represented by a vector $\overrightarrow{m}_{ref}^{test}$ and further values $m_{ref}$ which belong to medical samples whose spectra are assigned to the validation data set are represented by a $\overrightarrow{m}_{ref}^{val}$. Thereby the assignment between medical sample, measured spectra and reference value $m_{ref}$ is maintained.

Deriving the predictive model

A first predictive model to calculate a vector $\overrightarrow{m}_{pred,1}^{train}$ is generated by bioinformatic processing using the spectra of the training data set, wherein the bioinformatic processing comprises the steps of

a. Selection of n frequencies; with $n \in N$;
b. Calculating optimal weighting values for the frequencies selected;
c. Calculating the vector $\overrightarrow{m}_{pred,1}^{train}$;

In *step a.* a limited number of n frequencies $f_n$, with $n \in N$ is selected from the set of pre-processed spectra of the training data set which are suspected to contain the most information about a given value $m_{ref}$. According to the invention 5 to 1000 frequencies $f_n$ are selected from the set of pre-processed spectra of the training data set, preferably 5 to 100 frequencies $f_n$ are selected from the set of pre-processed spectra of the training data set, most preferably 5 to 50 frequencies $f_n$ are selected from the set of pre-processed spectra of the training data set.

It is important to note, that frequencies which are selected must not belong to resonances in the spectra belonging directly to a certain molecule and e.g. its concentration, but can also be an indirect indicator of complex physical and physiological processes in the sample or the patient reflected not only by a single molecule concentration.

In one embodiment of the invention the selection of the frequencies is arbitrary. In a further embodiment of the invention the frequencies are selected by a pre-selection to find the best candidates. For example, the correlation of the frequencies with the marker can be checked and frequencies with the highest correlation are taken.

It has to be noted that the term frequency applies to nearly all spectra types. However, in the case of Mass-Spectroscopy it translates to mass. For the sake of clarity, the mass returned by mass spectroscopy is subsumed as 'frequency' in the context of the present invention.

The selected frequencies are represented by the spectral vector $\overrightarrow{s_f} = \begin{pmatrix} s_1(f) \\ s_2(f) \\ \cdot \\ \cdot \\ \cdot \\ s_n(f) \end{pmatrix}$ for one frequency $f$ and $n$ medical samples. The selected frequencies are represented by the spectral matrix $S$ which considers the number of medical samples $n$ and the total number of selected frequencies $k$ which were selected for the spectra available for each medical sample.

$$S = \begin{pmatrix} s_1(f_1) & \cdots & s_1(f_k) \\ \vdots & \ddots & \vdots \\ s_n(f_1) & \cdots & s_n(f_k) \end{pmatrix}.$$

Accordingly, a column of spectral matrix **S** represents the spectral vector $\vec{s}_f$ for one frequency *f* of *n* medical sample, while a row contains one or more spectra of a medical sample. More accurately, every row $s_i$ represents a medical sample *i*, for which *l* spectra were measured. For each of the spectra the defined number of frequencies are extracted:

$$S = \begin{pmatrix} s_1^{(1)}(f_1^{(1)}) & \cdots & s_1^{(1)}\left(f_{k(1)}^{(1)}\right) & s_1^{(2)}\left(f_1^{(2)}\right) & \cdots & s_1^{(l)}(f_{k(l)}^{(l)}) \\ \vdots & \ddots & & & \vdots \\ s_n^{(1)}(f_1^{(1)}) & \cdots & & & & s_n^{(l)}(f_{k(l)}^{(l)}) \end{pmatrix}.$$

$s_1^{(l)}\left(f_{k(l)}^{(l)}\right)$ would denote the last value of spectrum *l* measured for sample 1. For the sake of simplicity, we stay with the notation of the first definition.

The spectral matrix **S** can be linearly expanded to contain also non-linear terms:

$$S = \begin{pmatrix} s_1(f_1) & \cdots & s_1(f_k) & s_1(f_1) \cdot s_1(f_1) & \cdots & s_1(f_k) \cdot s_1(f_k) & \frac{s_1(f_2)}{s_1(f_1)} & \cdots & \frac{s_1(f_k)}{s_1(f_{k-1})} \\ \vdots & \ddots & & & \vdots & & \\ s_n(f_1) & \cdots & s_n(f_k) & s_n(f_1) \cdot s_n(f_1) & \cdots & s_n(f_k) \cdot s_n(f_k) & \frac{s_n(f_2)}{s_n(f_1)} & \cdots & \frac{s_n(f_k)}{s_n(f_{k-1})} \end{pmatrix}.$$

This way of expansion can be solved in the same linear way and uses the same frequencies as in the linear case. In one embodiment of the invention in a first step only linear terms are used to optimize the weighting. Higher order terms such as second order product and fraction are only used for optimization for frequencies that are found to improve the prediction using linear terms only.

$$\vec{w} = \begin{pmatrix} w_1 \\ w_2 \\ \cdot \\ \cdot \\ \cdot \\ w_k \end{pmatrix}$$

In *step b.* for the selection of frequencies a weight vector is established, which results in the vector of predicted values $\vec{m}_{pred} = S \cdot \vec{w}$, which contains a prediction for each marker. For the first predictive model derived the calculated vector $\vec{m}_{pred}$ is called $\vec{m}_{pred,1}^{train}$.

The weights $\vec{w}$ are optimized by standard algebraic methods to minimize the error between $m_{pred}$ and $\vec{m}_{ref}$.

The user of the method according to the invention can define for any kind of spectrum that is involved, how many of the n selected $f_n$ frequencies are considered by defining the weights which are non-zero. More frequencies or weights will generally improve the quality of the prediction, but also increase the danger of over-fitting.

Once the optimum weights for a set of frequencies represented by the spectral vector $\vec{S}$ or the matrix **S** is determined and a first predictive model *F(s)*, is obtained.

According to the invention the first predictive model is optimized. A quality score $d_1^{train}$ is calculated utilizing the vectors $\vec{m}_{pred,1}^{train}$ and $\vec{m}_{ref}^{train}$. $d_1^{train}$ is the difference between the vectors $\vec{m}_{pred,1}^{train}$ and $\vec{m}_{ref}^{train}$ and is defined as $d_1^{train} = \left| \vec{m}_{ref}^{train} - \vec{m}_{pred,1}^{train} \right|$. The larger the difference $d_1^{train}$ the larger the error and accordingly the lower the quality of the prediction.

The first predictive model is utilized to calculate a vector $\vec{m}_{pred,1}^{test}$ using the spectra of the test data set and calculating a quality score $d_1^{test}$ utilizing $\vec{m}_{ref}^{test}$ and $\vec{m}_{pred,1}^{test}$, wherein $d_1^{test} = \left| \vec{m}_{ref}^{test} - \vec{m}_{pred,1}^{test} \right|$.

To generate an optimized predictive model a further predictive model to calculate a vector $\vec{m}^{train}_{pred,2}$ by bioinformatic processing is generated, comprising the steps

    a. Selection of n frequencies, with $n \in N$;
    b. Calculating optimal weighting values for the frequencies selected;
    c. Calculating the vector $\vec{m}^{train}_{pred,2}$.

Wherein the spectra of the training data set are used and wherein the same number n of frequencies is selected as in the first step. The n selected frequencies are iteratively altered compared to the frequencies selected in any of the steps before. Steps a. to c. of the bioinformatic processing are already explained in the context of deriving the first predictive model. Simultaneously to the steps performed to derive the first predictive model the spectra of the training data set were used, but the frequencies selected in step a. are iteratively altered compared to the frequencies selected for generating the first predictive model and to the frequencies selected in any of the steps before.

The quality score $d^{train}_2$ utilizing $\vec{m}^{train}_{ref}$ and $\vec{m}^{train}_{pred,2}$ is calculated, wherein $d^{train}_2 = \left| \vec{m}^{train}_{ref} - \vec{m}^{train}_{pred,2} \right|$. The further predictive model is utilized to calculate a vector $\vec{m}^{test}_{pred,2}$ using the spectra of the test data set and calculating a quality score $d^{test}_2$ utilizing $\vec{m}^{test}_{ref}$ and $\vec{m}^{test}_{pred,2}$, wherein $d^{test}_2 = \left| \vec{m}^{test}_{ref} - \vec{m}^{test}_{pred,2} \right|$.

The quality scores are compared and only if $d^{train}_2 < d^{train}_1$ and $d^{test}_2 < d^{test}_1$ the further predictive model is taken as optimal predictive model and the first predictive model is discarded. If the condition $d^{train}_2 < d^{train}_1$ and $d^{test}_2 < d^{test}_1$ is not fulfilled the first predictive model continuous to be taken as optimal predictive model and the further predictive model is discarded. To simplify the following procedure the quality scores of the optimal predictive model are renamed as $d^{train}_{opt}$ and $d^{test}_{opt}$, wherein $d^{train}_{opt}$ is the quality score calculated using the spectra of the training data set and $d^{test}_{opt}$ is the quality score calculated for the spectra of the test data set.

Further steps 10) to 12) of the method are repeated, thereby obtaining again a further predictive model. To obtain any further predictive model the spectra of the training data set are used, but the n frequencies selected in step 10) are iteratively altered compared to the frequencies selected for generating any predictive model before. The quality scores of the optimal predictive model and the further predictive model are compared and if $d^{train}_2 < d^{train}_{opt}$ and $d^{test}_2 < d^{test}_{opt}$ the further predictive model is taken as optimal predictive model. If the condition $d^{train}_2 < d^{train}_{opt}$ and $d^{test}_2 < d^{test}_{opt}$ is not fulfilled the optimal predictive model is unchanged and the further predictive model is discarded. The quality scores of the predictive model taken as optimal predictive model are renamed as $d^{train}_{opt}$ and $d^{test}_{opt}$ if necessary. Again, $d^{train}_{opt}$ is the quality score calculated using the spectra of the training data set and $d^{test}_{opt}$ is the quality score calculated for the spectra of the test data set.

As can be seen the immediate measure that drives the optimization process is the difference between the vectors $m_{ref}$ and $\vec{m}_{pred}$, which is mathematically described by the quality score d. Generating a further predictive model and comparing the quality scores as described to find the optimal predictive model is driven by the selection of frequencies.

Testing all combinations of frequencies in the spectra is computationally actually out of reach. According to the invention step 14) is repeated for all possible combinations of n frequencies. That means, while the number of frequencies selected is constant n, with $n \in N$, the n frequencies which are selected to generate a predictive model are iteratively altered for generating different predictive models.

In one embodiment of the invention two measures are taken to reduce computational effort without losing crucial information. First, the correlation of each frequency vector $\vec{s_f}$ with $\vec{m}_{ref}^{train}$ is calculated. Sorting by correlation allows selecting most relevant frequencies. Dependent on the spectra type 100 to 5000 frequencies are selected, preferably 200 to 2000 frequencies are selected, most preferably 500 to 1000 frequencies are.

The second measure is to split the search for combinations into two distinct phases. During the first phase all possible combinations are evaluated for a selected number of frequencies. Between 2 and 100 frequencies are selected, preferably between 3 and 20 frequencies are selected, most preferably between 5 and 10 frequencies are selected. As this is the most time-consuming part, the maximum number that is feasible depends on available resources.

In the second phase the remaining frequencies are evaluated linearly, by adding them only if they improve the score significantly. What significant means depends on the range of values of the marker. Assuming a normalized $\vec{m}_{ref}^{train}$ with values $-1 \leq \vec{m}_{ref,i}^{train} \leq 1,$ a significant improvement would mean in one implementation $1 \cdot 10^{-10}$ - $1 \cdot 10^{-1}$, in a preferred implementation $1 \cdot 10^{-5}$ - $1 \cdot 10^{-2}$.

For non-normalized markers the significant minimum improvements are multiplied by the range of values of the marker.

Evaluating the optimal predictive model

In the next step the quality of the predictive model to predict a certain value $m_{ref}$ or a vector $\vec{m}_{ref}$ is evaluated.

According to the invention $\vec{m}_{pred}^{val}$ is calculated with the pre-processed spectra of the validation data set, which was not involved in generating the predictive model, utilizing the optimal predictive model as independent test. Afterwards, the correlation coefficient of $\vec{m}_{pred}^{val}$ and $\vec{m}_{ref}^{val}$ is calculated. In one embodiment of the invention this is done using the Pearson, Spearman, Kendall or other correlation coefficient. This has the advantage, of helping the user to understand the quality of the predictive model in an easy way. Furthermore, especially when working with spectra that are not normalized, the correlation coefficient helps to compare the quality for different predictive models and also the quality of the prediction between different types of markers.

During the process of deriving the predictive model questionable results could be derived due to the noise in the spectra and/or due to overfitting. Since the predicting model has a certain complexity there is also a certain likeliness to reach a high-quality measure, meaning a high correlation coefficient by chance. For example, if the number of latent variables, meaning the number of non-zero weights, exceeds the number medical samples used to derive the predicting model it becomes very likely that one is able to predict anything within the spectra used to derive the predicting model. However, such a model is unlikely to predict anything meaningful if used in the method according to the invention. Therefore, according to the invention a z-score is calculated using the spectra of the validation data set to evaluate the trustworthiness of the correlation coefficient.

The z-score utilizes a random distribution of $\vec{m}_{ref}^{random}$ as reference. For a given number n of spectra and a given number k of frequencies f a random distribution for the $\vec{m}_{ref}^{random}$ is created and a predictive model is derived by the method according to the invention as described. $m_{pred}$ values are calculated with the predictive model and the correlation coefficient $q_{rand}$ is calculated. This process is repeated several thousand times and the resulting correlation coefficients $q_{rand}$ are collected.

From the distribution of $q_{rand}$ values both their mean value $\bar{q}_{rand}$ as well as the standard deviation $\sigma_{rand}$ are calculated. With this the significance of the correlation coefficient $q_{pred}$ calculated for $\vec{m}_{pred}^{val}$ can be estimated as:

$$z = \frac{q_{pred} - \bar{q}_{rand}}{\sigma_{rand}}$$

If the correlation coefficient $q_{pred}$ of the calculated $\overrightarrow{m}^{val}_{pred}$ is one standard deviation better than the average random quality, this would result in a z-score of 1. Up to a z-score of ~2 there is a significant chance to obtain a given quality measure by chance - which would render the value more or less meaningless. The larger the z-score, the more reliable a given quality measure can be considered.

Hence, a cross validation is performed since the spectra of the validation data set are used to validate the optimal predictive model. The spectra of the validation data sat are not involved in the steps of generating the optimal predictive model and represent therefore an independent data set. The predictive model is considered to be stable and thus overfitting is excluded if the correlation coefficient is higher or equal 0.7 and if the z-score is larger than 2. In this case the predictive model is considered to have a good enough quality for a reliable prediction of a certain marker. Thus, in one embodiment of the invention the correlation coefficient of each optimal predictive model is equal to or higher than 0.7 and the z-score of each optimal predictive model is higher than 2.

In one embodiment of the invention the correlation coefficient is higher than or equal 0.7, preferably higher than or equal 0.8, most preferably higher than or equal 0.9. Wherein predicting models with a correlation coefficient higher than or equal 0.9 can be considered as very good, predicting models with a correlation coefficient higher than or equal 0.8 can be considered as good and predicting models with a correlation coefficient higher than or equal 0.7 can be considered as satisfactory.

If overfitting is detected by calculating the correlation coefficient the z-score, the number of latent variables, meaning the number of non-zero weights is reduced. In this way the number of frequencies exploited to build the predicting model is reduced. If non-linear terms were included, their number could be also reduced prior to reduce the overall number of frequencies.

If multiple spectra are used per medical sample, the number of non-zero weights can be defined individually for every type of spectra. This gives the opportunity to define the relative importance of every type of spectrum. Therefore, it is possible to take into account to what degree a further spectrum can add new information to an already existing model.

Accordingly, if the predicting model derived by a certain spectral matrix S does not predict values $\overrightarrow{m}^{val}_{pred}$ with a satisfying quality, the following can be done:

- using an alternative way of pre-processing of the measured spectra
- add additional spectra with different preprocessing
- selecting other frequency components of the spectra for the spectral matrix
- allowing a different number n of frequencies, wherein $n \in N$.

During pre-processing especially noise filtering and normalization can be done in different ways or can even be left undone. In one embodiment of the invention pre-processing steps are performed in a different way compared to the pre-processing done to derive the predictive model which has an unsatisfying quality. The newly pre-processed set of spectra is again used to derive a predictive model by the steps already described.

Further, a new predictive model is derived by the steps explained above, wherein other frequency components are selected for the spectral vector or the spectral matrix. The pre-processing of the measured spectra can be left unchanged or can be done in a different way compared to the pre-processing done to derive the predictive model which has an unsatisfying quality.

These steps are repeated until a predictive model is obtained which is able to calculate the value $m_{pred}$ for a given $m_{ref}$ with a satisfying quality. Accordingly, a predictive model is obtained iteratively to calculate a certain value $m_{pred}$ which is a prediction for a value $m_{ref}$.

In one embodiment of the invention after deriving a satisfying predictive model, the relative importance of every non-zero weighting factor $w_i$ is estimated by checking how much the calculated value $m_{pred}$ changes when switching this weighting factor to zero. In this way the number of free variables can be reduced by identifying weight factors $w_i$ which are not necessary to derive a satisfying predictive model for a certain marker. Thereby, the risk of overfitting can be further reduced.

According to the invention a predictive model is obtained in this way for every marker, which shall be predicted by the method according to the invention. Hence, a multiple number of predictive models is generated, wherein every predictive model is suited to calculate a value $m_{pred}$ which is a prediction for a certain marker.

Further, the generated predictive models are used in the method according to the invention to predict markers which are characteristic for at least one medical sample and/or for a patient comprising the steps:

- Passing at least one medical sample to at least one spectrometer by a user;

- Measuring at least one spectrum of the at least one medical sample with at least one spectroscopic method;
- Calculating multiple values $m_{pred}$ with the at least one spectrum of the at least one medical sample using multiple predictive models;
- Classifying the calculated values $m_{pred}$ in terms of defined thresholds;
- Creating a report including recommended actions for the user;
- Initiating further diagnostic steps by the user;

wherein the at least one medical sample is examined in an untargeted manner;
wherein the values $m_{pred}$ are predictions of markers, which are characteristic for said at least one medical sample and/or a patient; and
wherein each value $m_{pred}$ is calculated by a separate predictive model.

The bioinformatic processing to generate the predictive model comprises pre-processing of the utilized spectra, frequency selection, weight optimization, estimation of a quality measure and detecting and correction of overfitting. In a preferred embodiment of the invention pre-processing is also done with the at least one spectrum used for calculating multiple $m_{pred}$ values according to the method of the invention. Pre-processing is done in the same way as already described above for deriving the predicting model.

Accordingly, calculation of each value $m_{pred}$ for a medical sample is performed based on n measured spectra s by

$$m_{pred} = F(\tilde{s}),$$

wherein $\tilde{s}$ are the one or more spectra for a medical sample, while F is the predictive model. Latter comprises the selection of the frequencies (including non-linear terms) and the multiplication with the weight-vector. The timeframe required to operate the method according to the invention is determined by the transport of the medical sample and by the time needed to measure the spectra. Advantageously, the calculation of a multiple of $m_{pred}$ values is very fast.

Further the calculated values $m_{pred}$ are classified in terms of defined thresholds. According to the invention the thresholds to classify the calculated values $m_{pred}$ are defined using $m_{pred}$ and $m_{ref}$ values utilized in the method of generating the predictive model.

In many cases a binary classification is useful to illustrate the result of the method for a user in an easily understandable way. Binary classification means a certain value or a certain statement like "healthy" or "having a certain disease". If the reference value $m_{ref}$ is binary as well, this is inherently obtained. Which means by comparing $m_{ref}$ and $m_{pred}$ values a threshold can be defined for $m_{pred}$, which separates two defined statuses (e.g. "healthy" and "having a certain disease").

When the $m_{ref}$ values are continuous or have a range of values, such a classification can be obtained in terms of a threshold for the calculated value $m_{pred}$ and thus for the marker. A suitable threshold for a certain marker can be derived utilizing the $m_{pred}$, $m_{ref}$ pairs used to generate the predictive model. Therefore, $m_{pred}$, $m_{ref}$ pairs are presented in a confusion matrix, containing true positive, false positive, true negative and false negative values. By plotting the development of the confusion values (e.g. false positives) for a range of possible thresholds it is possible to determine the ideal threshold, leading to the best separation. It should be noted, that the meaning of best separation and therefore the ideal threshold depends on the character of the marker. For markers relating to critical conditions (e.g. risk of mortality) one likely prefers not to miss true positives and accepts a higher false positive rate. For markers relating not to such critical conditions one probably wants to reduce both false rates. Therefore, the threshold for a certain marker is determined individually for the marker and hence can advantageously be adapted to the characteristics of the marker. Accordingly, the classification of the calculated values according to the invention enables the classification of the calculated values and therefore of the predicted markers in terms of reference intervals and/or cut-offs and/or probabilities of diagnoses, events and medical complications, which supports the clinical assignment and the initiation of the further necessary treatment steps.

According to the invention, a report is created which includes recommended actions for the user. In one embodiment the report includes the marker predicted by the calculated values $m_{pred}$ optionally in context with the values of conventional routine laboratory medical diagnostic if available and/or a classification.

In one embodiment of the invention the markers predicted by the calculated values $m_{pred}$ are compared with results of conventional routine laboratory medical diagnostic and/or interpreted in the context of a classification, describing reference intervals and/or cut-offs and/or probabilities of diagnoses, events and medical complications. In one embodiment of the invention a part of the medical sample is therefore examined in parallel or prior to performing of the method according to the invention by targeted routine laboratory medical diagnostics. In a further embodiment of the invention the medical sample is examined by targeted routine laboratory medical diagnostics after performing the method according to the invention. Since the medical sample may change some of its properties due to storage conditions or aging effects, additional information can be obtained depending on the point in time the routine laboratory medical diagnostics is performed.

In a preferred embodiment the report makes clearly visible significant differences between markers predicted by the calculated values $m_{pred}$ and values obtained by conventional routine laboratory medical diagnostic or the exceeding of a threshold for example by a color code. The exceeding of a threshold of a marker predicted by a calculated value $m_{pred}$ and/or differences between markers predicted by the calculated values $m_{pred}$ and values obtained by conventional routine laboratory medical diagnostic can also be made visible and/or transmitted to the laboratory information system and/or physician/pathologist to trigger re-measurements or other medical procedures.

Due to the comparison of the markers predicted by the calculated values $m_{pred}$ and the values obtained by conventional routine laboratory medical diagnostic or by classifying the calculated values further actions are recommended to the user. If a difference between markers predicted by the calculated values $m_{pred}$ and the values obtained by conventional routine laboratory medical diagnostic is detected it will be recommended to the user to check the results measured by routine laboratory medical diagnostic and if suitable to re-measure certain values. Accordingly, with the invention it is possible to identify and prevent invalid results measured by routine laboratory medical diagnostic and thus a quality check of these results is performed in an easy and fast way.

Analogues, due to the markers like diagnosis, events or complications predicted by the calculated values $m_{pred}$, further actions are recommended to the user. Further actions include further diagnostic steps such as re-measuring certain values measured by conventional routine laboratory medical diagnostic, detailed medical validation of the medical report, blocking or withdrawal of the results and reports, propose further diagnostic steps to complement the values already obtained, exclusion of sample confusion, request for a new patient's sample and consultation with the patient and/or sample sender. The user reviews the recommendations given and takes any further diagnostic steps or action that seem necessary.

Since the method according to the invention is based on measuring at least one spectrum obtained by a spectroscopic method the medical sample is examined in an untargeted manner. Which means many different markers such as laboratory values, clinical features and sample characteristics are measured simultaneously. Advantageously, a user has not to decide beforehand for a suspected diagnosis and the appropriate diagnostic laboratory medical parameters which have to be evaluated.

Accordingly, in one embodiment of the invention between 10 and 1000 $m_{pred}$ values are calculated, preferably between 20 and 500 $m_{pred}$ values are calculated, most preferably between 50 and 250 $m_{pred}$ values are calculated. Wherein one calculated value $m_{pred}$ is used to predict one certain marker.

In one embodiment of the invention the method comprises the step of permanently further optimizing the predictive model. Since during performing the method according to the invention spectra are measured for new medical samples as well as routine laboratory diagnostic is performed for these medical samples according to one embodiment of the invention, additional data is available to improve the predictive model. In one embodiment of the invention the continuous improvement of the prediction model will be performed by repeating the same procedure as described for deriving the predictive model, using the growing database (meaning the larger number of medical samples available). In another implementation learning algorithms will be trained to improve the given predictions. Updating of the predictive model can be performed both locally (no transfer of patient related data) or anonymized and encrypted on a server.

Since the frequencies selected for the predictive model do not necessarily belong directly to a certain molecule and e.g. its concentration but can also be an indirect indicator of a complex situation reflected not only by a single molecule concentration, it is possible to calculate values $m_{pred}$ which are predictions of concentrations of molecules in the medical sample with a concentration below the detection limit of the spectroscopic methods used to measure the at least one spectrum. For instance, if troponin T is elevated in blood in case of myocardial infarction, it has been released from damaged heart muscle cells. Analogous to troponin other molecules have also been released by these cells und are now elevated in the blood sample. Some of these molecules are already used as additional biomarkers for myocardial infarction as myoglobin, troponin I and heart muscle specific creatine kinase. However, there are many more molecules released by the damaged cells as Heart-type Fatty Acid-Binding Protein, orthophosphate d-glucosyltransferase, S100A0 and others. These and further biomarkers as ischemia-modified albumin or Copeptin also show a correlation with troponin as they belong to corresponding metabolic networks and are all elevated when heart muscle cells are damaged [Aydin, S Vasc Health Risk Manag. (2019)]. As the application of the method allows to detect many different biomarkers in parallel, the correlation between biomarkers allow to estimate the concentration of the desired biomarker, in this case troponin, although this cannot be measured directly with sufficient accuracy by conventional Raman spectrometry or another described methods.

This feature broadens the application of the method and provides useful additional information for a user. Further, this is a feature which is not provided by routine laboratory diagnostics.

In one embodiment of the invention at least one marker in the medical sample can be predicted due to relationships in metabolic networks, even if it is not detectable in the sample with routine laboratory methods.

If the predictive model is permanently further optimized it is possible to obtain more $m_{pred}$ values or maybe $m_{pred}$ values having a better quality, meaning higher precision and therefore are a more precisely prediction of a certain marker. In this case, it can be useful to repeat the method according to the invention with a set of spectra which was already treated by the method before to compare the obtained information or to complement these. Therefore, in one embodiment of the invention at least one spectrum of a medical sample of each spectroscopic method used is permanently saved.

In a further of the invention the medical sample is passed to the at least one spectrometer on a foil tape. In this embodiment the medical sample is applied on a foil tape and the foil tape is passed to the at least one spectrometer. In a preferred embodiment several medical samples are spaced from each other applied on a foil tape, thereby the same foil tape can be used to investigate several medical samples. Further, a substance used as control and/or calibration substance is also applied on the foil tape. The control and calibration substance allows mechanical adjustment of the measuring equipment and verification of the measuring conditions such as the laser power, frequency, detector precision and detector sensitivity. In addition, the calibration substance allows additional preprocessing of the spectra of the medical samples, e.g. normalization and specific background correction. With this application the precision and validity of the prediction can be improved and spectrometer defects as well as measurement problems can be identified immediately. This minimizes the risk of incorrect diagnostic results.

Further, the invention provides a device to perform the method steps

- Passing a sample to at least one spectrometer by a user;
- Measuring at least one spectrum of the sample with at least one spectroscopic method;
- Calculating multiple values $m_{pred}$ which are predictions of values characteristic for said sample by bioinformatic processing of at least one spectrum of the using multiple predictive models;
- Comparing the calculated values with results of conventional routine laboratory medical diagnostic and/or comparing the predicted values with reference intervals;
- Creating a report including recommended actions for the user;
- Initiating further diagnostic steps by the user;

comprising

- At least one spectrometer;
- At least one data processing device;
- At least one user interface.

All features described for the method according to the invention apply also for the device according to the invention and vice versa.

The at least one spectrometer is suited to measure the at least one spectrum of a medical sample. Therefore, suitable spectrometers are selected from the group containing Raman spectrometer, MR-spectrometer, UV-spectrometer, NIR-spectrometer, VIS-spectrometer, TR-spectrometer and mass spectrometer.

The device comprises at least one data processing device which can be a PC or a tablet. The data processing device should be suited to perform the bioinformatic processing according to the invention.

Further, the device comprises at least one user interface. Such user interface can be a touchscreen of a PC or a tablet, also suitable is a touchscreen of a smartphone. The interface should be suitable to display the report created by the method of the present invention.

In one embodiment the device further comprises a foil tape. In a further embodiment of the invention the device comprises a foil tape and optionally a CCD-camera. The foil tape is used to transport at least one medical sample to at least one spectrometer. Preferably the foil tape transports more than one medical sample to more than one spectrometer. The foil tape can further comprise at least one substance for calibration and/or control purposes. Optionally a CCD camera investigates the medical samples and/or the substance for calibration and/or control purposes by performing an air bubble check and/or a drop evaluation. This is necessary because, despite highly standardized application of the medical samples, artifacts such as air bubbles cannot be reliably excluded and further information as the color of the sample can be detected. In addition, the drop shape can influence the maximum height of the liquid column as well as the optical properties. In these cases, the spectra of the samples can either be normalized accordingly or the measurement of the sample has to be repeated.

In one embodiment the foil tape is wound on a roll, from which it is unwound and guided over several rolls. In this manner a medical sample and/or a substance for calibration and/or control purposes can be transported to several spectrometers and/or CCD-cameras. The foil tape preferably comprises a material which does not interact with any kind of medical sample. In one embodiment of the invention the foil tape comprises Polyethylene (PE).

Further, the method according to the invention or the device according to the invention can be used for validity and quality check of conventional targeted routine laboratory medical diagnostic, for performing untargeted diagnostics, to obtain a rapid diagnosis of suspicion and/or to for monitoring of laboratory values and/or clinical features of a patient at home, in a doctor's practice or in the clinic.

Rapid diagnosis of suspicions could be useful for general practitioners to have a hint which further diagnostic steps should be initiated. Further since some spectroscopic methods can already be performed from untrained persons it is possible that the method and the device according to the invention are performed by patients at home. In this case patients are enabled to monitor laboratory values and/or clinical features regularly. Results can be on the one hand reported to the general practitioners and on the other hand a warning can be given to the patient to consult a doctor if a certain marker is predicted which is above a certain threshold.

The method and the device according to the invention provide several advantages compared to the state of the art:

- The untargeted examination of medical samples is enabled by the method of the present invention. A multiple of values $m_{pred}$ is calculated by the method which are predictions of certain markers. Therefore, it is not necessary that a clinician initiates examination of the medical sample for certain values on the basis of a certain suspected diagnosis. Additionally, markers which are characteristic for the medical sample and/or the patient will be reported which can give hints to a clinician which diagnosis could also be possible and which further diagnostic should be applied.
- $m_{pred}$ values can serve to perform a quality check of a parallel or prior performed routine laboratory diagnostic and also allows the identification of samples with faulty preanalytics.
- Parallel analysis of spectra measured with different spectroscopic methods is enabled. Accordingly, information contained in different spectra measured with different spectroscopic methods can complement each other and can deliver more precise and a higher number of $m_{pred}$ values and thus of markers.
- Additionally, parallel analysis of different medical samples of one patient can be investigated. This opens up the possibility to combine the information contained in different medical samples and further obtain information which are only available due to the combination of information contained in spectra of different medical samples of one patient.
- It is possible to calculate $m_{pred}$ values which predict the concentration of molecules in the medical solution which is below the detection limit of the spectroscopy methods used to obtain the spectra used to calculate the $m_{pred}$ values.
- Not only concentrations of certain molecules can be predicted by the calculated $m_{pred}$ but also clinical markers and events, patient's sex and age, clinical diagnosis, medical complications, progress of a disease, risk of mortality, intake of drugs and food supplements, intoxications, drug interactions, and sample characteristics such as sample-age.
- Many of above factors, such as sample-age, drugs and food supplements, impact routine diagnostics. The knowledge about them, as provided by the invention, will significantly improve the quality of routine diagnostics.
- Just small amounts of medical sample volume are sufficient to perform the method of the present invention.
- Since spectroscopic methods are already broadly available and no further complex mechanization compared to routine analyzers is necessary the method and the device of the invention are cost-efficient and highly economical.
- Further in comparison to standard targeted laboratory diagnostics less reagents and other consumables are needed and therefore costs can be saved.

In the following the invention is further described by 7 figures and 4 examples.

Figure 1  illustrates the correlation coefficient of several markers for different predictive models using the training data set;

Figure 2  illustrates the correlation coefficient of several markers for different predictive models using the test data set;

Figure 3  illustrates the correlation coefficient of several markers for different predictive models using the validation data set;

Figure 4  A and B illustrate the correlation of reference values and predicted markers;

Figure 5  A and B illustrate the correlation of reference values and the predicted markers;

Figure 6  illustrates the application of the present invention parallel to standard routine diagnostics;

Figure 7  (A) illustrates an embodiment of the invention, wherein a medical sample is applied to a foil tape and (B) illustrates the surface of a foil tape.

For a cohort of 403 patients serum samples were collected. Further, five Raman spectra were collected for serum sample, using an excitation frequency of 758 nm. As all patients were investigated in the context of cirrhosis of the liver, relevant biomarkers markers were measured by routine measuring methods of a clinical laboratory. Serum of patients with end-stage liver disease presents a difficult substrate for Raman-spectroscopy, since there is an accumulation of different metabolites that cause high fluorescence signals that suppress the Raman-signal. However, the results described below shows impressively the advantages and opportunities of the method of the present invention. The following table 1 gives an overview of liver related markers:

Table 1

| Liver related biomarkers | |
|---|---|
| Albumin (ALB_S) | Serum albumin as a liver synthesis parameter |
| International normalized ratio of prothrombin time (INRVOR_C) | Clotting test (Prothrombin time as INR) dependent of coagulation factors synthesized by the liver. This parameter is measured in another material (citrate plasma) and not measurable in the analyzed material by routine diagnostics. |
| Cholinesterase (CHE_S) | A liver synthesis parameter. Due to its very low concentrations in serum, the enzyme activity in routine diagnostics depends on the detection of substrate turnover while this invention allows direct approximation of the activity. |
| Bilirubin (BILI_S) | Liver function and detoxification parameter in serum |
| alanine amino- transferase (ALAT_S) | Results of enzymatic tests indicating liver damage. Due to their very low concentrations in serum, the enzyme activity in routine diagnostics depends on the detection of substrate turnover while this invention allows direct approximation of the activity. |
| Aspartate aminotransferase (ASAT_S) | |
| $\gamma$-Glutamyltranspeptidase (GGT_S) | |
| Total cholesterol (CHOL_S) | Total serum cholesterol. Reduced serum concentrations are often detected in patients with end-stage liver disease |
| Alpha fetoprotein (AFP_S) | Tumor marker for the detection of hepatocellular cancer. Alpha fetoprotein concentration in serum is usually very low and routine laboratory diagnostics has to be based on highly sensitive immunological detection. |
| **Clinical features of patients** | |
| Hemodialysis (HAEMODIA) | Hemodialysis status of the patients. Not detectable with routine diagnostic methods (0=no, 1=yes). |
| Hepatocellular carcinoma (HCC) | Hepatocellular status of the patients. Not detectable with routine diagnostic methods (0=no, 1=yes). |
| **Other Biomarkers of clinical relevance** | |
| Creatinine (CRE_S) | Most important renal function parameter in serum. |
| c-reactive protein (CRP_S) | An indicator of infections and or inflammation in the patients. Very low serum concentrations. |
| Sodium (NA_S_fusio) | Sodium serum concentration |
| platelets (B-PLT_E) | Number of platelets in EDTA whole blood. However, concentration was approximated by analyzing serum, where platelets are not measurable with routine methods |
| **Prognostic Scores for end-stage liver disease** | |
| MELDNA_UNOS (MELD Score including sodium) | International established prediction scores for the estimation of 3-month mortality of patients with end-stage liver disease, e.g. for prioritization of liver transplant, the score incorporates the results of bilirubin, creatinine, INR and hemodialysis-status, sodium optional. |
| MELD_ber (MELD score without sodium) | |
| **Pre-analytical Quality Indicators** | |
| Hemolysis index (INDH_S) | Laboratory medical indicator of hemolysis |
| Icterus-Index (INDI_S) | Laboratory medical indicator of icterus |
| Lipemia Index INDL_S | Laboratory medical indicator of lipemia |

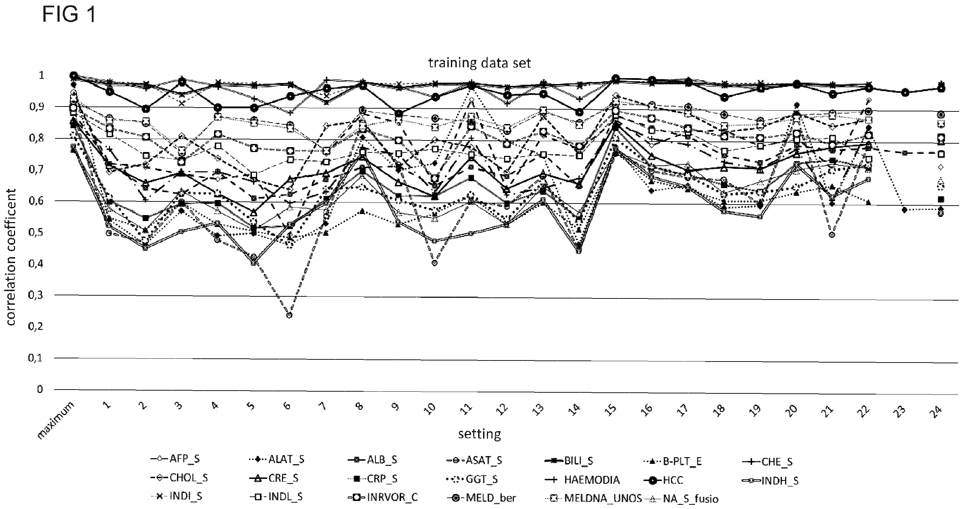

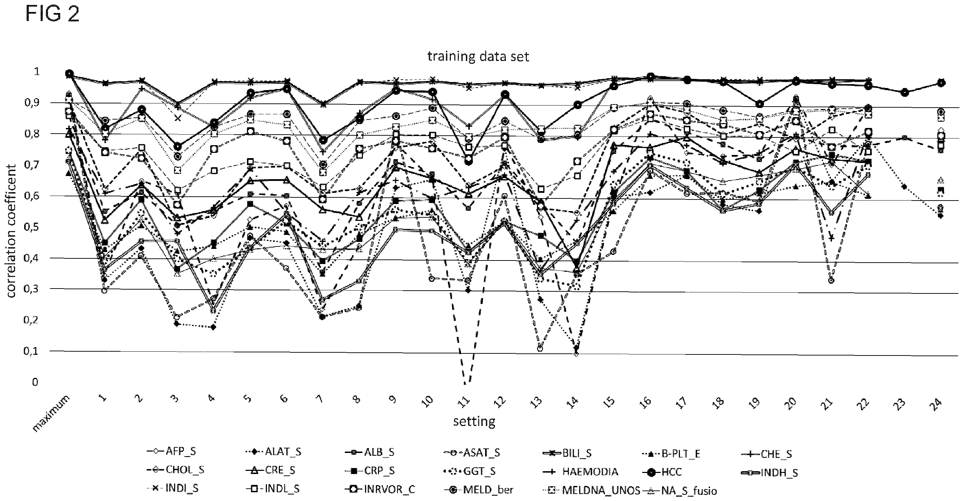

The samples were divided into three subgroups: the train data, test data and validation data set. The training data set is driving the optimization primarily as described above. **Figure 1** illustrates the correlation coefficient of several markers for different predictive models using the training data set. Different predictive models are labelled as setting numbers on the x axis. The test data set is used as verification during the optimization and is therefore integral part of the optimization as well. The correlations of test and training data set indicate the information content which can be expected from a dataset. Ideally, predictions would reach a similar level of correlation. **Figure 2** illustrates the correlation coefficient of the same markers for the same predictive models as shown in figure 1 using the test data set.

Both figures show correlation coefficients obtained for the training data set and the test data set using different predictive models. Thereby, the figures plot the correlation coefficients with reference data (represented by laboratory diagnostics, medical scores, clinical features) as y-axis and the different predictive models used as x-axis. Although values for different predictive models of the same marker are connected for convenience, it has to be noted that there is no continuity in a mathematical sense between different points on the x-axis.

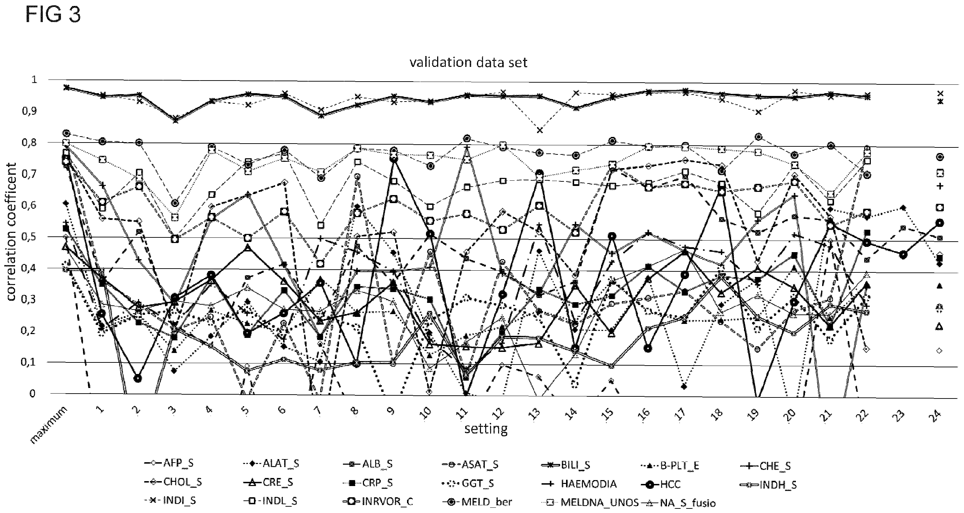

Further, **figure 3** illustrates the correlation coefficient of several markers for different predictive models using the validation data set. The validation data set is an independent data set not involved in the optimization, its correlation with the reference data is therefore indicating the actual predictive power.

One can compare the correlation coefficients obtained for the training data set, the test data set and the validation data set for one predictive model. Larger differences between correlation coefficients of training data set and validation data set indicate that the optimization is not perfect yet, or in other words the full predictive power is not reached yet.

Reasons for lower correlation coefficients for the validation data set than the training data set can be due to:

- Not the right predictive model found yet
- A lack of data (e.g. if the number of available laboratory reference data varies between markers)
- overfitting

It should be noted that the method of the present invention has an inherent tendency to find best solutions and overfitting which could occur is counterbalanced by an averaging of weights determined in different iterations.

It can be seen from figures 1 to 3 that a number of markers show just small changes upon different predictive models. For those markers predictive models are found which with a high quality.

However, it is noteworthy that some of the markers show significant peaks. And that different markers have these peaks for different predictive models. On the one hand this shows that different predictive models provide different correlation coefficients, as expected. Thus, for many markers the optimal predictive model which reveals the full predictive potential will only be obtained by certain preprocessing steps according to the invention. On the other hand, it is clear that optimization of the predictive model has to be performed for each marker individually.

Clinical results and benefits from the intervention

- The combination of two different spectrometric principles (Raman- and Fluorescence spectroscopy) in addition with different physical effects (photometric bleaching), re-measurements (5 spectra of each samples) and different mathematical calculations (e.g. mean, differences, deviations) between the spectra resulted in complex information about the sample and provided a robust basis for the approximation of markers which represent medical parameters, scores and/or clinical features of the patients.
- The invention provides the reagents free parallel approximation results of >15 laboratory medical parameters, scores and clinical features of the patients. The number of parameters is only limited by the information supplied in the clinical dataset.
- All results can be achieved from only 50$\mu$l of sample. Routine diagnostics require different materials and higher volumes due to sequential measurements.
- The result of the International Normalized Ratio and with a lower precision the platelets of the patient can be approximated from measurement of serum, although these parameters are not measurable from serum with standard laboratory methods.
- Complex medical scores e.g. the MELD scores can easily be approximated with the invention from one sample with good accuracy. In contrast for conventional measurement and calculation of MELD-Diagnostic hemodialysis of the patients has to be known. This is no longer necessary.
- Low concentrated analytes that cannot be directly measured in the samples by spectroscopic methods (in this case e.g. AFP and CRP by Raman spectroscopy) became predictable by machine learning, complex informatics and statistical calculations.
- Diagnostic of enzymes in blood samples is elementary for clinical diagnostic but requires clinical chemical methods with measurement of substrate turnover. The present invention provides the prediction of different enzyme activities.

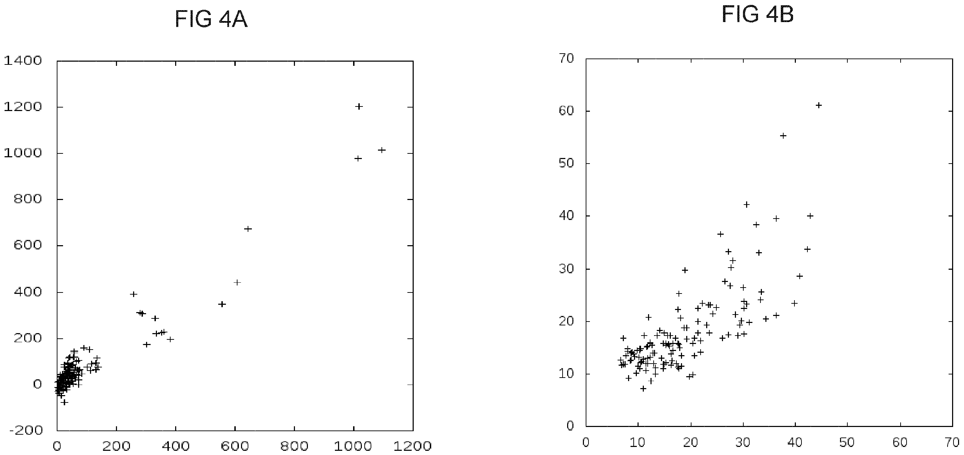

**Figure 4 (A)** illustrates the correlation between the reference values of BILI_S and the values predicted by the method according to the invention for this marker. The y-axis shows the reference values and the x-axis the values predicted by the method according to the invention. Best correlation is given if the data-points are approximating a diagonal line, worst correlation and thus a bad quality of the method of the invention would result in a broadly distributed scatter plot. Further, **figure 4 (B)** illustrates the correlation between the reference values of the MELD score and the values predicted by the method according to the invention for this marker.

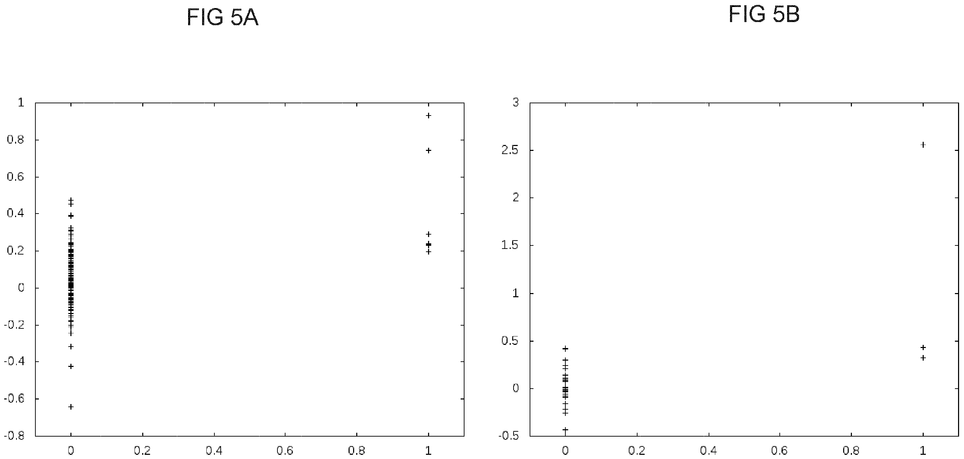

**Figure 5 (A)** illustrates the correlation between the reference values of HAEMODIA and the values predicted by the method according to the invention for this marker. HAEMODIA is the Hemodialysis which corresponds to a status of a patient. Reference values cannot be obtained with standard routine laboratory diagnostics, but has to be entered by the attending physicians and is checked against the dialysis protocols of the patient. Since HAEMODIA has no numerical representation per se, the information of this marker is translated into numerical values. 0 is equivalent to the status: no HAEMODIA, and 1 is equivalent to the status: yes HAEMODIA. As can be seen a threshold of the value on the y-axis of the marker becomes evident at which the status of the HAEMODIA switches from 0 to 1. For HAEMODIA the threshold is between 0.2 and 0.5. Figure 5 (B) illustrates the correlation between the reference values of HCC and the values predicted by the method according to the invention for this marker. HCC indicates whether the patient is suffering from hepatocellular carcinoma. This is not directly detectable with standard routine laboratory diagnostics, but usually has to be diagnosed on the basis of different imaging and sometimes tumor biopsy. Like HAEMIDIA HCC has no numerical representation per se, therefore the information of this marker is translated into numerical values. 0 is equivalent to the status: no HCC, and 1 is equivalent to the status: yes HCC. A threshold at which the status changes can be clearly seen in figure 5 (B) and is between 0.4 and 0.5.

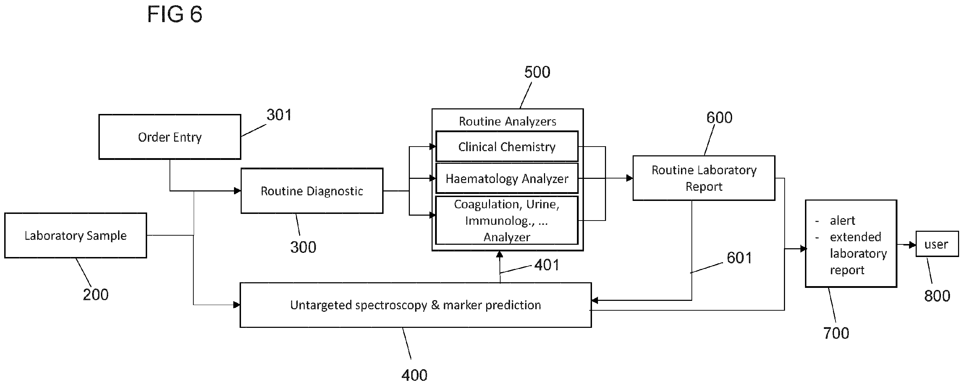

**Figure 6** illustrates the application of the present invention parallel to standard routine diagnostics. In this embodiment a laboratory sample is provided 200 and a batch of 50μl of the medical sample is provided to be investigated by the method according to the invention 400 while the rest of the sample is provided to be investigated by standard routine diagnostics 300. For standard routine diagnostics an order entry 301 is required which requests a number of certain laboratory values. Based on the order entry 301 the sample is investigated by corresponding routine standard diagnostic methods 500. In contrast the batch of 50μl provided to be investigated by the method according to the invention 400 is investigated independent of any order entry since the sample is investigated by untargeted spectroscopic methods according to the invention. Advantageously, standard routine diagnostic steps and the investigations of the sample according to the invention can be processed in parallel. Results of standard routine diagnostics are analyzed by routine analyzers 500 such as clinical chemistry, haematology analyzer, coagulation analyzer, urine analyzer, immunology analyzer and so on. A routine laboratory report 600 is provided by standard routine diagnostics. If there are significant discrepancies between targeted and non-targeted diagnostics 401, the results of routine diagnostics should be critically reviewed to rule out incorrect diagnostics. There may be problems with the measuring instruments or the sample material. Re-measurement of controls can help to identify corresponding problems of routine instruments. Problems with the sample material can be due to pre-analytical influencing and interfering factors. For example, blood coagulation may have led to insufficient sample aspiration or the patient may have taken medication that may interfere with routine methods. Consequently, diagnostic errors with possible medical consequences can be avoided.

The standard laboratory report can further more provide a further data base to improve the predictive models according to the invention by a feedback loop 601. If the routine laboratory report or the markers predicted by the invention give a hint that a potential live threatening condition is probable an immediate alert is given to a user like a technician or a medical doctor. Furthermore, the results of the routine laboratory report and the markers predicted by the method of the invention are combined in an extended laboratory report 700. This provides several advantages, first the markers predicted by the method of the present invention can give a hint that other laboratory values except of those which were already requested by the initial order entry should be investigated by standard routine diagnostics. Further the quality of the laboratory values measured by standard routine diagnostics can be evaluated by comparing predicted markers and laboratory values measured by standard routine diagnostics. Therefore, the report is given to a user 800 like a technician or a medical doctor which is able to initiates further investigations by standard laboratory diagnostics based on the markers predicted by the present invention and on the laboratory values measured. Accordingly, the extended laboratory report 700 includes for example recommendations for further diagnostic proceedings and identification of invalid results.

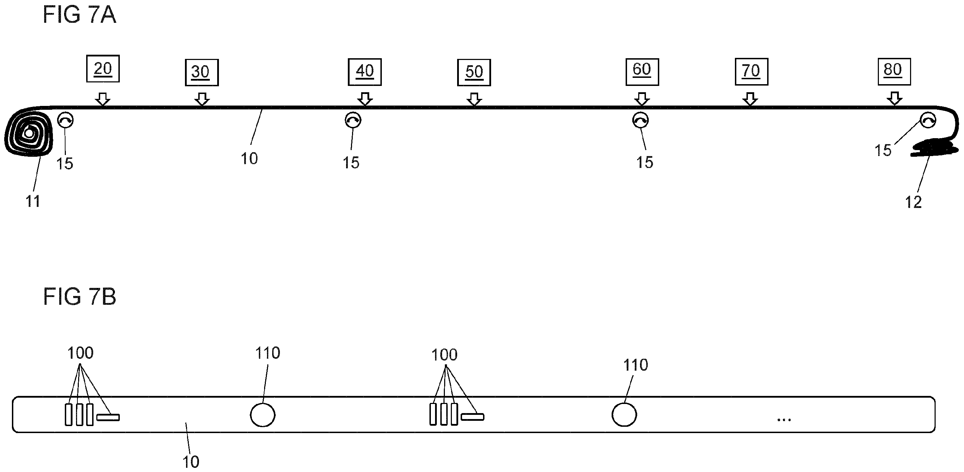

**Figure 7 (A)** illustrates an embodiment of the invention, wherein a medical sample 20 is applied to a foil tape 10. The foil tape 10 can be wound on a roll 11, from which it is unwound and guided over several rolls 15. The foil tape preferably comprises a material which does not interact with any kind of medical sample. In one embodiment of the invention the foil tape comprises Polyethylene (PE).

The sample is applied to the foil tape 10 in step 20 and afterwards in a first investigation step 30 the sample can be investigated by a CCD-camera to perform an air bubble check and drop evaluation. In a next investigation step 40 multiple measurements with spectroscopic methods like NIR-, VIS- and/or UV-spectroscopy can be performed. Further in step 50 multiple Raman spectroscopy measurements can be performed. Optionally in step 60 and step 70 spectroscopic measurements analogous to steps 40 and 50 can be performed. This has the advantage, that spectroscopic measurements from different time points can be provided. The chance of the results of the spectroscopic methods due to bleaching and other physical processes adds additional information to the predicted markers. In a further embodiment in steps 60 and 70 different spectroscopic methods compared to steps 40 and 50 are applied to the medical sample. Combination of different spectroscopic methods will also add new information to the predicted markers as already explained. After investigating the medical sample with spectroscopic methods in step 80 the medical sample is again investigated by a CCD-camera to perform a final drop evaluation, which can be used as process control parameter. The foil tape is finally given to a waste container 12.

In a further embodiment of the invention not only a medical sample 110 is applied to the foil tape 10 but also a calibration or control substance 100. The medical sample 110 and the calibration or control substance 100 can be applied alternately as shown in **figure 7 (B)** or the calibration or control substance 100 can be applied after a certain number of medical samples have been applied, e.g. after 2 to 10 medical samples have been applied to the foil tape 10. The calibration or control substance 100 can serve for example to calibrate the x-y-position on the surface of the foil tape to make sure that the medical samples 110 are positioned in the right manner to apply spectroscopic methods.

**Example 1 - Parallel untargeted analysis in parallel to standard targeted routine diagnostic**

A patient with fatigue, abdominal pain and jaundice visits an emergency outpatient clinic. Routinely only EDTA-plasma and serum are collected and the standard laboratory diagnostic is requested: blood count, liver and renal parameters from blood serum. As the transaminases are increased a hepatic problem is suspected. The patient is referred to a hepatological outpatient clinic and is given an appointment for a presentation in one week.

According to the invention, in parallel to the routine diagnostic 50$\mu$l of blood serum of the patient is processed automatically on a Raman spectrometer by a user and Raman spectra were measured at 785 nm, Laser power level: 150.0 mW, Filter: 785 nm, Grating: 400 lines/mm, Spectrograph aperture: 50 $\mu$m slit; 3 times with exposure time: 2.00 sec and Number of exposures: 5 and 2 times with exposure time: 3.00 sec and number of exposures: 10.

After digital processing of the Raman spectra serum levels and fluorescence background laboratory parameters, scores and clinical features were estimated from the trained prediction models. According to the invention, it turned out that the MELD score was increased, indicating a dramatically elevated three-month mortality. MELD score could be calculated although International Normalized Ratio (INR) not measurable with standard laboratory diagnostics from serum, bilirubin was not requested and the dialysis status was not specified. The emergency outpatient clinic is immediately informed about the serious clinical suspicion. The patient is then contacted and admitted to the hospital to initiate the treatment without delay.

In summary, the invention accelerated the diagnosis and therapy of the patient, by also being able to predict results from blood serum when the necessary laboratory medical materials are not available. By the untargeted character of this diagnostics also not requested laboratory biomarkers could be predicted and reported, to ensure in-time the necessary medical consequences.

**Example 2 - usage of the investigation by a patient at home**

A device according to the invention comprising a compact and easy to handle Raman spectrometer was given to a patient to monitor certain markers once a week. The patient gave 50$\mu$l of urea once a week inside the spectrometer and a Raman spectrum was measured at a Raman frequency of 785 nm. All measurement settings were fixed before the device was given to the patient. A number of 30 $m_{pred}$ values were calculated using 30 optimal predictive models, wherein every $m_{pred}$ value was a prediction of one certain marker. The calculated values $m_{pred}$ were classified in terms of defined threshold and a report was created. The report was on the one hand saved to be controlled by a general practitioner later on and on the other hand the report was shown to the patient.

The protein per creatinine and leucocytes were above a certain defined threshold. Since this indicates a recurrence of a previously diagnosed disease (e.g. vasculitis with kidney involvement), the patient was given the recommendation by the report to visit a doctor at the same day.

**Example 3 - usage of the investigation by general practitioner**

A device according to the invention comprising a compact and easy to handle Raman spectrometer was given to a family doctor to pre-analyse serum of potential critical patients. General practitioners usually cooperate with a clinical laboratory that performs laboratory diagnostic for the patients. Patient's samples were collected usually once a day. This delays laboratory diagnostic for some patients.

From the sample 50$\mu$l of serum are placed inside the spectrometer. The investigation allows to collect a Raman spectrum at 785nm. All measurement settings were fixed before the device was given to the patient. A number of 30 $m_{pred}$ values were calculated using 30 optimal predictive models, wherein every $m_{pred}$ value was a prediction of one certain marker. The procedure takes about 3 minutes. The calculated values $m_{pred}$ were classified in terms of defined threshold and a report was created. The report was on the one hand saved to be controlled by the clinical laboratory later on and on the other hand the report immediately allows the general practitioner a preliminary more accurate clinical assessment of the patient. In addition, the laboratory diagnostics can be extended according to medical requirements. This allows an accelerated further process and could improve patient's safety.

**Example 4 - urine measurement**

Urine diagnostic is elementary for laboratory diagnostic. Actually, clinical diagnostic is based on urine test strips and spectroscopic methods are not used in routine diagnostics due to complex patient-individual different compositions of urine.

The invention provides the possibility to estimate the analytes concentrations by reagent-free diagnostics based the method according to the invention. 921 different urine samples were measured using Raman spectroscopy, each sample was measured five times (Spectrometer: DXR SmartRaman, Laser: 785 nm, Laser power level: 150.0 mW, Filter: 785 nm, Grating: 400 lines/mm, Spectrograph aperture: 50 $\mu$m slit; 3 times with exposure time: 2.00 sec and Number of exposures: 5 and 2 times with exposure time: 3.00 sec and number of exposures: 10.)

Glucose (QU-GLU_U) is used as the primary parameter to illustrate the optimization of the prediction method. At the beginning only one of the five measurements of each sample is used as training data set to build a predictive model. Fluorescence background is filtered at first. Without filtering of fluorescence background, no predictive model could be established.

At the beginning there is only a weak correlation between the glucose urine concentration of the routine diagnostic (dip stick test) and the predictive model of the invention.

QU-GLU_U correlation of validation data set: 0.561217

After including all five spectra of each sample in the calculation the results increased to: QU-GLU_U correlation of validation data set: 0.664297

After including differences of the spectra in the calculation, the results further improved. Finally, the correlation reached >0.8 after further inclusion mean result of the spectra to calculate a predictive model: QU-GLU_U correlation of validation data: 0.806942

Each time the number of predictive values had to be balanced for best results. Higher numbers lead to better results in the training dataset but the results of the test and more significantly the results of the validation data decreased.

It has to be pointed out, that the dip stick results from routine diagnostics are of limited validity and therefore higher correlations are not realistic, e.g. dip stick tests only present semi quantitative results with a limited accuracy and are susceptible to a variety of drug interactions as ascorbic acid and other antioxidants.

Conclusions, clinical results and benefits

- Filtering of background fluorescence is essential for the creation of a predictive model even for urine diagnostic.
- A continuous improvement of the predictive performance of the framework became possible due to optimization of the input information.
- Precise predictions required an input of repetitive measurements, inclusion of differences between these measurements as well as calculation of mean values.
- The resulting predictive model seems to be adequate to routine dipstick diagnostic without any need of reagents.
- Other diagnostic parameters as pH, protein, ketones, blood, bilirubin, urobilinogen, nitrite, leucocytes and specific gravity could be included with the same specific optimization procedure. However, each analyte requires specific parameters to achieve adequate correlations.
- The number of parallel parameters is limited by the training data set.
- Inclusion of different spectrometric methods has the potential for further improvement of the accuracy of the prediction even of low concentrated analytes.
- Dependent on the dataset also other patient's characteristics and clinical features could be predicted.

**Citations**

Regan M., Forsman R. (2006): The impact of the laboratory on disease management, Dis Manag. 2006 Apr; 9(2):122-30

Makary, Martin A.; Daniel, Michael (2016): Medical error-the third leading cause of death in the US. In: BMJ (Clinical research ed.) 353, i2139. DOI: 10.1136/bmj.i2139

Rohleder et al. (2004): Quantitative analysis of serum and serum filtrate by means of Raman spectroscopy, The Analyst, 129, 906-911

Plebani M. (2006): Errors in clinical laboratories or errors in laboratory medicine?, Clin Chem Lab Med (CCLM), Band 44, Heft 6. DOI: https://doi.org/10.1515/CCLM.2006.123

Aydin, S Vasc Health Risk Manag. (2019): 15: 1-10. Published online 2019 Jan 17. doi: 10.2147/VHRM.S 166157

**Claims**

1. Method to predict markers which are characteristic for at least one medical sample and /or for a patient comprising the steps:

    • Passing at least one medical sample to at least one spectrometer by a user;
    • Measuring at least one spectrum of the at least one medical sample with at least one spectroscopic method;
    • Calculating multiple values $m_{pred}$ with the at least one spectrum of the at least one medical sample using multiple optimal predictive models;
    • Classifying the calculated values $m_{pred}$ in terms of defined thresholds;
    • Creating a report including recommended actions for the user;
    • Initiating further diagnostic steps by the user;

    wherein the at least one medical sample is examined in an untargeted manner;
    wherein the values $m_{pred}$ are predictions of markers, which are characteristic for said at least one medical sample and/or a patient; and
    wherein each value $m_{pred}$ is calculated by a separate predictive model.

2. Method according to claim 1 **characterized in that** the optimal predictive model to calculate a value $m_{pred}$ or a vector $\vec{m}_{pred}$ is generated by a model based or a model-free approach.

3. Method according to any of the preceding claims **characterized in that** the optimal predictive model to calculate a value $m_{pred}$ or a vector $\vec{m}_{pred}$ is generated by a method comprising the steps:

    1) Passing at least 100 medical samples to at least one spectrometer by a user;
    2) Measuring at least one spectrum of each of the at least 100 medical samples with at least one spectroscopic method;
    3) Pre-processing all spectra;
    4) Reading a value $m_{ref}$ which represents a reference value for $m_{pred}$ for every of the at least 100 medical samples;
    5) Dividing all of the pre-processed spectra into a training data set, a test data set and a validation data set;
    6) Representing the values $m_{ref}$ which belong to the training data set by a vector $\vec{m}_{ref}^{train}$, the values $m_{ref}$ which belong to the test data set by a vector $\vec{m}_{ref}^{test}$ and the values $m_{ref}$ which belong to the validation data set by a vector $\vec{m}_{ref}^{val}$;
    7) Generating a first predictive model to calculate a vector $\vec{m}_{pred,1}^{train}$ by bioinformatic processing using the spectra of the training data set, wherein the bioinformatic processing comprises the steps of

        a. Selection of n frequencies, with $n \in N$;
        b. Calculating optimal weighting values for the frequencies selected;
        c. Calculating the vector $\vec{m}_{pred,1}^{train}$;

    8) Calculating a quality score $d_1^{train}$ utilizing $\vec{m}_{ref}^{train}$ and $\vec{m}_{pred,1}^{train}$;

9) Utilizing the first predictive model to calculate a vector $\vec{m}_{pred,1}^{test}$ using the spectra of the test data set and calculating a quality score $d_1^{test}$ utilizing $\vec{m}_{ref}^{test}$ and $\vec{m}_{pred,1}^{test}$;

10) Generating a further predictive model to calculate a vector $\vec{m}_{pred,2}^{train}$ by bioinformatic processing comprising the steps

    a. Selection of n frequencies, with $n \in N$;
    b. Calculating optimal weighting values for the frequencies selected;
    c. Calculating the vector $\vec{m}_{pred,2}^{train}$; using the spectra of the training data set, wherein the n frequencies selected in step a. are iteratively altered compared to the frequencies selected in any of the steps before;

11) Calculating a quality score $d_2^{train}$ utilizing $\vec{m}_{ref}^{train}$ and $\vec{m}_{pred,2}^{train}$;

12) Utilizing the further predictive model to calculate a vector $\vec{m}_{pred,2}^{test}$ using the spectra of the test data set and calculating a quality score $d_2^{test}$ utilizing $\vec{m}_{ref}^{test}$ and $\vec{m}_{pred,2}^{test}$;

13) Comparing the quality scores and only if $d_2^{train} < d_1^{train}$ and $d_2^{test} < d_1^{test}$ the further predictive model is taken as optimal predictive model and the first predictive model is discarded; if the condition $d_2^{train} < d_1^{train}$ and $d_2^{test} < d_1^{test}$ is not fulfilled the first predictive model is taken as optimal predictive model and the further predictive model is discarded; and renaming the quality scores of the optimal predictive model as $d_{opt}^{train}$ and $d_{opt}^{test}$;

14) Repeating steps 10) to 12) and comparing the quality scores of the optimal predictive model and the further predictive model and only if $d_2^{train} < d_{opt}^{train}$ and $d_2^{test} < d_{opt}^{test}$ the further predictive model is taken as optimal predictive model; if the condition $d_2^{train} < d_{opt}^{train}$ and $d_2^{test} < d_{opt}^{test}$ is not fulfilled the optimal predictive model is unchanged and the further predictive model is discarded; and renaming the quality scores of the optimal predictive model as $d_{opt}^{train}$ and $d_{opt}^{test}$ if necessary;

15) Repeating step 14) for all possible combinations of n frequencies;

16) Utilizing the optimal predictive model to calculate a vector $\vec{m}_{pred}^{val}$ using the spectra of the validation data set;

17) Validating the optimal predictive model by calculating a correlation coefficient and a z-score utilizing the vector $\vec{m}_{pred}^{val}$;

wherein the values of the vector $\vec{m}_{pred}^{train}$ are predictions of the values of the vector $\vec{m}_{ref}^{train}$, the values of the vector $\vec{m}_{pred}^{test}$ are predictions of the values of the vector $\vec{m}_{ref}^{test}$ and the values of the vector $\vec{m}_{pred}^{val}$ are predictions of the values of the vector $\vec{m}_{ref}^{val}$.

**4.** Method according to one of the preceding claims, **characterized in that** the correlation coefficient of each optimal predictive model is equal to or higher than 0.7 and the z-score of each optimal predictive model is higher than 2.

**5.** Method according to one of the preceding claims, **characterized in that** the thresholds to classify the calculated values $m_{pred}$ are defined using $m_{pred}$ and $m_{ref}$ values utilized in the method of generating an optimal predictive model.

**6.** Method according to one of the preceding claims, **characterized in that** the method further comprises the step of measuring respectively at least one spectrum of the medical sample by 1 to 7 further spectroscopic methods, preferably by 1 to 4 further spectroscopic methods.

**7.** Method according to one of the preceding claims, **characterized in that** the spectroscopic methods are selected from the group containing Raman spectroscopy, surface enhanced Raman spectroscopy (SERS), plasmon-enhanced Raman spectroscopy (PERS), including surface-enhanced Raman spectroscopy (SERS), shell-isolated nanoparticle-enhanced Raman spectroscopy (SINERS) and tip-enhanced Raman spectroscopy (TERS), near infrared spectroscopy (NIR), ultraviolet spectroscopy (UV), visible spectroscopy (VIS), terahertz spectroscopy (TR), magnetic resonance spectroscopy (MR) and mass spectroscopy.

**8.** Method according to one of the preceding claims, **characterized in that** the markers which are predicted by the calculated values $m_{pred}$ are selected from the group comprising laboratory values, clinical features and sample characteristics.

**9.** Method according to one of the preceding claims, **characterized in that** the medical sample is selected from the group comprising blood, blood plasma, blood serum, cerebrospinal fluid (CSF), urine and saliva.

**10.** Method according to one of the preceding claims, **characterized in that** a part of the medical sample is examined in parallel, prior to or after performing the method according to claim 1 by targeted routine laboratory medical diagnostic.

**11.** Method according to one of the preceding claims **characterized in that** said method further comprises the step of further optimizing the predictive model.

**12.** Method according to one of the preceding claims **characterized in that** at least 10 $m_{prep}$ values are calculated.

**13.** Method according to one of the preceding claims, **characterized in that** at least one marker with a concentration in the medical sample below the detection limit of at least one of the spectroscopy methods used is predicted.

**14.** Method according to one of the preceding claims, **characterized in that** at least one marker in the medical sample can be predicted due to relationships in metabolic networks, even if it is not detectable in the sample with routine laboratory methods.

**15.** Method according to one of the preceding claims, **characterized in that** the medical sample is passed to the at least one spectrometer on a foil tape.

**16.** Device adapted to perform the method steps

- Passing at least one medical sample to at least one spectrometer by a user;
- Measuring at least one spectrum of the at least one medical sample with at least one spectroscopic method;
- Calculating multiple values $m_{pred}$ with the at least one spectrum of the at least one medical sample using multiple optimal predictive models;
- Classifying the calculated values $m_{pred}$ in terms of defined thresholds;
- Creating a report including recommended actions for the user;
- Initiating further diagnostic steps by the user;

comprising

◦ At least one spectrometer;
◦ At least one data processing device;
◦ At least one user interface.

**17.** Device according to step 15, **characterized in that** the device further comprises a foil tape, and optionally a CCD-camera.

## FIG 1

training data set

EP 3 971 909 A1

## FIG 2

training data set

FIG 3

EP 3 971 909 A1

FIG 4A

FIG 4B

EP 3 971 909 A1

FIG 5A

FIG 5B

EP 3 971 909 A1

FIG 6

FIG 7A

| 20 | 30 | 40 | 50 | 60 | 70 | 80 |

15   10   15   15   15

11   12

EP 3 971 909 A1

FIG 7B

100   110   100   110

10

...

Europäisches
Patentamt

European
Patent Office

Office européen
des brevets

# EUROPEAN SEARCH REPORT

Application Number

EP 20 19 7251

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/173390 A1 (APPLIED PROTEOMICS INC [US]) 5 October 2017 (2017-10-05) * paragraphs [0010], [0011], [0070], [0099], [0114], [0114], [0021] * ----- | 1-17 | INV. G16H50/20 |
| A | US 2014/349337 A1 (DASARI RAMACHANDRA [US] ET AL) 27 November 2014 (2014-11-27) * paragraphs [0036] - [0038] * ----- | 1-17 | |
| A | US 2019/316203 A1 (DAVISON TIMOTHY [GB] ET AL) 17 October 2019 (2019-10-17) * paragraphs [0271], [0249], [0342], [0252] * ----- | 1-17 | |
| A | "33rd Annual Meeting & Pre-Conference Programs of the Society for Immunotherapy of Cancer (SITC 2018)", JOURNAL FOR IMMUNOTHERAPY OF CANCER, BIOMED CENTRAL LTD, LONDON, UK, vol. 6, no. 1, 6 November 2018 (2018-11-06), pages 1-205, XP021262326, DOI: 10.1186/S40425-018-0422-Y * page 21, paragraph 41 * ----- | 1-17 | |
| A | WO 2007/126901 A2 (CANGEN BIOTECHNOLOGIES INC [US]; MOON CHULSO [US]) 8 November 2007 (2007-11-08) * the whole document * ----- | 1-17 | TECHNICAL FIELDS SEARCHED (IPC) G16H |
| A | CN 103 364 389 A (SHANGHAI FOOD & DRUG TESTING INST) 23 October 2013 (2013-10-23) * the whole document * ----- | 1-17 | |

The present search report has been drawn up for all claims

| Place of search | Date of completion of the search | Examiner |
|---|---|---|
| Munich | 9 February 2021 | Laub, Christoph |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 20 19 7251

This annex lists the patent family members relating to the patent documents cited in the above-mentioned European search report.
The members are as contained in the European Patent Office EDP file on
The European Patent Office is in no way liable for these particulars which are merely given for the purpose of information.

09-02-2021

| Patent document cited in search report | | Publication date | Patent family member(s) | | Publication date |
|---|---|---|---|---|---|
| WO 2017173390 | A1 | 05-10-2017 | CN | 109416360 A | 01-03-2019 |
| | | | EP | 3436831 A1 | 06-02-2019 |
| | | | US | 2019113520 A1 | 18-04-2019 |
| | | | WO | 2017173390 A1 | 05-10-2017 |
| US 2014349337 | A1 | 27-11-2014 | US | 2014349337 A1 | 27-11-2014 |
| | | | WO | 2013096856 A1 | 27-06-2013 |
| US 2019316203 | A1 | 17-10-2019 | AU | 2016295347 A1 | 22-02-2018 |
| | | | CA | 2993142 A1 | 26-01-2017 |
| | | | CN | 108138236 A | 08-06-2018 |
| | | | EP | 3325653 A1 | 30-05-2018 |
| | | | JP | 2018524990 A | 06-09-2018 |
| | | | US | 2019316203 A1 | 17-10-2019 |
| | | | WO | 2017013436 A1 | 26-01-2017 |
| WO 2007126901 | A2 | 08-11-2007 | EP | 2010895 A2 | 07-01-2009 |
| | | | KR | 20090012313 A | 03-02-2009 |
| | | | US | 2008046224 A1 | 21-02-2008 |
| | | | WO | 2007126901 A2 | 08-11-2007 |
| CN 103364389 | A | 23-10-2013 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Aydin, S Vasc Health Risk Manag,* 2019
- **REGAN M. ; FORSMAN R.** The impact of the laboratory on disease management. *Dis Manag,* April 2006, vol. 9 (2), 122-30
- **MAKARY, MARTIN A. ; DANIEL, MICHAEL.** Medical error-the third leading cause of death in the US. *BMJ (Clinical research ed.),* 2016, vol. 353, i2139

- **ROHLEDER et al.** Quantitative analysis of serum and serum filtrate by means of Raman spectroscopy. *The Analyst,* 2004, vol. 129, 906-911
- **PLEBANI M.** Errors in clinical laboratories or errors in laboratory medicine?. *Clin Chem Lab Med (CCLM),* 2006, vol. 44, 6, https://doi.org/10.1515/CCLM.2006.123
- *Aydin, S Vasc Health Risk Manag,* 17 January 2019, vol. 15, 1-10